# EUROPEAN PATENT APPLICATION

(11) **EP 2 581 743 A1**
(43) Date of publication of application: **17.04.2013**
(21) Application number: 13150002.7
(22) Date of filing: 02.08.2007
(51) Int. Cl.: G01N 33/53, C07K 16/18

(54) **Method and reagent for detecting chronic liver disease by measurement of autotaxin**

(30) Priority: 03.08.2006 JP 2006212275; 30.03.2007 JP 2007092412
(62) Divisional of application: 07792235.9
(71) Applicant: The University of Tokyo, Bunkyo-ku, Tokyo 113-8654 (JP); TOSOH CORPORATION, Shunan-shi Yamaguchi 746-8501 (JP)
(72) Inventor: Aoki, Junken, Tokyo, 113-8654 (JP); Arai, Hiroyuki, Tokyo, 113-8654 (JP); Yatomi, Yutaka, Tokyo, 113-8654 (JP); Ikeda, Hitoshi, Tokyo, 113-8654 (JP); Nakamura, Kazuhiro, Tokyo, 113-8654 (JP); Igarashi, Koji, Kawasaki-shi, Kanagawa 215-0003 (JP); Ide, Kazufumi, Kawasaki-shi, Kanagawa 214-0021 (JP)
(74) Representative: Pautex Schneider, Nicole Véronique

(57) **Abstract**

A method for detecting chronic liver disease is provided comprising : measuring the concentration of autotaxin in a human specimen by an immunochemical measurement method using antibody, and judging to be a chronic liver disease in the case that the measured value exhibits a significantly higher value than normal values comprised of measured values from normal healthy subjects.

## Description

### TECHNICAL FIELD

The present invention relates to an antibody that specifically recognizes and binds naturally-occurring form of human autotaxin, a screening method thereof, and a method and reagent for detecting malignant lymphoma based on measurement of autotaxin concentration in a specimen.

### BACKGROUND ART

Human autotaxin is a glycoprotein having a molecular weight of about 125 kDa first isolated from a culture medium of A2058 human melanoma cells by M.L. Stracke, et al. in 1992 as a substance that induces cell motility (J. Biol. Chem., 256, 2524, 1992). Subsequently, as a result of an analysis of its structure by J. Murata, et al. in 1994 using cDNA cloning, it was determined to have a single transmembrane moiety on the amino terminal, contain two somatomedin B regions, i.e., type I phosphodiesterase activity and an EF hand loop regions in the extracellular domain, and have phosphodiesterase activity (J. Biol. Chem., 269, 30479, 1994). However, there was yet to be any well-defined evidence acquired relating to the function of autotaxin of inducing cell motility. In 2002, new lysophospholipase D was identified in human (A. Tokumura, et al., J. Biol. Chem., 277, 39436, 2002) and bovine serum (M. Umezu-Goto, et al., J. Cell. Biol., 158, 227, 2002), thereby demonstrating for the first time that lysophosphatidic acid formed by this enzyme activity induces cell motility.

Based on an inference from the motility-inducing function of autotaxin, Stracke, et al. described the use of autotaxin as a marker for the invasive capabilities of human cancer in US Patent No. 5,449,753 (1995). In addition, Stracke, et al. also suggested a cancer therapy by administration of anti-autotaxin antibody bound to toxin in US Patent No. 5,731,167 (1998). In this manner, although there is a desire for an antibody that specifically recognizes human autotaxin, since autotaxin is contained at a relatively large amount in various forms in animal serum, it is expected to be extremely difficult to acquire antibody having specificity and strong binding strength to autotaxin that has not been subjected to mutation normally present in serum (naturally-occurring form of autotaxin). In actuality, there is no method for quantifying autotaxin per se, and is currently quantified indirectly by assessment of the lysophospholipase D activity it possesses. The procedure for measuring the activity of this enzyme is complex and normally requires an incubation time with substrate of several hours in order to allow the enzyme to demonstrate activity. In addition, there are also interrupting factors such as the likelihood of the presence of other enzymes having lysophospholipase D activity differing from autotaxin in human specimens, and the presence of lysophosphatidic acid and choline, formed by decomposition of the lysophosphatidyl choline substrate during measurement of enzyme activity, in human specimens. These intrinsic factors have at least some effect on specific quantification of human autotaxin during measurement of enzyme activity, and there is as of yet no highly accurate method for quantifying human autotaxin. In actuality, choline is contained in human plasma at a concentration on the order of several tens of mM, and it is difficult to measure activity in plasma based on the amount of choline formed using lysophosphatidyl choline for the substrate without pretreatment in the form of choline removal.

A large number of monoclonal antibodies have been established by several groups including ours by immunizing with soluble human autotaxin expressed by *Escherichia coli* using synthetic partial peptides and partial peptides of human autotaxin (Journal of Biological Chemistry, 269, 30479-30484, 1994, FEBS Letters, 571, 197-204, 2004). However, although human autotaxin can be detected by Western blotting using these antibodies, these antibodies do not exhibit any reactivity or only exhibit extremely low levels of reactivity with naturally-occurring form of human autotaxin present in human specimens. Accordingly, it has been extremely difficult to apply these antibodies to immunoassay methods such as ELISA for use with human specimens, and they have yet to be used practically.

According to the present invention, monoclonal antibody that specifically reacts with naturally-occurring form of human autotaxin in human specimens can be established extremely efficiently. Namely, in a process which screens out monoclonal antibodies prepared by use of antigen immunization and cell fusion steps, by selecting an antibody using as an indicator thereof reactivity with naturally-occurring form of antigen present in a solution, a human autotaxin quantifying system based on versatile method such as ELISA can be established. In addition, antibodies acquired by this method are high-performance antibodies enabling not only removal of antigen from a sample, but also antigen purification, by efficiently binding and capturing human autotaxin contained in blood and the like.

### DISCLOSURE OF THE INVENTION

Although there have been reports suggesting that concentrations at which human autotaxin is present in human tissues or body fluids fluctuate due to various diseases, since there has heretofore been no method for quantifying human autotaxin, detailed analyses thereof have not been performed. Although qualitative analyses have been conducted on the presence or absence of autotaxin and the ratios at which it is present under denaturing conditions such as in the presence of a reducing agent, since there has yet to be an antibody capable of efficiently recognizing and binding naturally-occurring form of human autotaxin normally present in the body while retaining its structure and function, a versatile method for quantifying human autotaxin has yet to be established. In addition, although analyses have been conducted on the causative relationship between disease and fluctuations in lysophospholipase D activity as determined by measurement of enzyme activity using as an indicator thereof the lysophospholipase D activity possessed by human autotaxin, since measured values obtained in this measurement of enzyme activity include lysophospholipase D activity other than that of autotaxin contained in human specimens, and since the lysophosphatidyl choline substrate used during measurement of activity as well as the choline formed during measurement of enzyme activity are intrinsically contained in specimens, these methods do not have enough reliability for use as true methods for specifically quantifying autotaxin.

We found a technique for efficiently acquiring antibody to naturally-occurring form of human autotaxin, and used the antibody to the naturally-occurring form of human autotaxin acquired with this method to successfully provide a method and reagent enabling accurate quantification of human autotaxin that is not affected by intrinsic substances that cause problems during measurement of activity and does not require pretreatment for denaturing the sample such as by denaturing with reduction treatment or denaturing using a protein denaturant such as guanidine hydrochloride or urea.

More specifically, the present application includes the following inventions:
(1) a method for screening antibody specifically recognizing naturally-occurring form of human autotaxin in a state in which it is present in the body without being denatured, comprising the steps of:
   binding a binding factor capable of capturing a candidate antibody of the antibody to a solid phase;
   binding the candidate antibody of the antibody to the binding factor;
   allowing naturally-occurring form of human autotaxin to react on a system in which the candidate antibody has reacted; and
   selecting an antibody that specifically recognizes the naturally-occurring form of human autotaxin by using as an indicator thereof the binding strength of the naturally-occurring form of human autotaxin to the antibody;
(2) the method of (1), wherein the binding factor is an antibody;
(3) the method of (1) or (2), wherein the naturally-occurring form of human autotaxin is a recombinant human autotaxin antigen having a polyhistidine tag;
(4) the method of (3), wherein the binding strength of the naturally-occurring form of human autotaxin to the antibody is measured by using a labeled anti-polyhistidine antibody that specifically binds to the recombinant human autotaxin antigen having the polyhistidine tag;
(5) the method of (4), wherein the labeled anti-polyhistidine antibody is an enzyme-labeled anti-polyhistidine antibody;
(6) an antibody specifically recognizing naturally-occurring form of human autotaxin in the state in which it is present in the body without being denatured capable of being acquired by a method comprising the steps of:
   binding a binding factor capable of capturing a candidate antibody of the antibody to a solid phase;
   binding the candidate antibody of the antibody to the binding factor;
   allowing naturally-occurring form of human autotaxin to react on a system in which the candidate antibody has reacted; and
   selecting an antibody that specifically recognizes the naturally-occurring form of human autotaxin by using as an indicator thereof the binding strength of the naturally-occurring form of human autotaxin to the antibody;
(7) the antibody of (6), wherein the binding factor is an antibody;
(8) the antibody of (6) or (7), wherein the naturally-occurring form of human autotaxin is a recombinant human autotaxin antigen having a polyhistidine tag;
(9) the antibody of (8), wherein the binding strength of the naturally-occurring form of human autotaxin to the antibody is measured by using a labeled anti-polyhistidine antibody that specifically binds to the recombinant human autotaxin antigen having a polyhistidine tag; and
(10) the antibody of (9), wherein the labeled anti-polyhistidine antibody is an enzyme-labeled anti-polyhistidine antibody.

According to the inventions as described above, a monoclonal antibody capable of specifically recognizing and binding naturally-occurring form of human autotaxin can be efficiently acquired, and a measurement method enabling quantification of autotaxin in a human specimen using this antibody can be constructed without requiring pretreatment and the like. Measurement of human serum using a naturally-occurring form of human autotaxin measurement reagent obtained according to the aforementioned inventions enables diagnosis of cancer or diagnosis of chronic liver disease. In addition, according to the aforementioned method, a method and reagent enabling quantification of human autotaxin can be provided that allows quantification to be carried out in a short period of time and without being affected by intrinsic measurement-inhibiting factors or competitive enzymes that conventionally caused problems in the estimation of autotaxin levels based on lysophospholipase D enzyme activity.

Moreover, the inventors of the present invention also found that human autotaxin contained in blood and the like can be quantified both easily and in a short period of time by using a versatile human autotaxin quantitative measurement system such as ELISA using a monoclonal antibody that specifically reacts with naturally-occurring form of human autotaxin present in a human specimen. As a result of conducting extensive studies using this measurement system, concentrations of autotaxin in serum were clearly determined to be high in patients with lymphomas, and particularly patients with follicular lymphomas. Malignant lymphomas are broadly categorized into two types consisting of Hodgkin's lymphomas and non-Hodgkin's lymphomas, and roughly 90% of the lymphomas in Japan are categorized as non-Hodgkin's lymphomas. Non-Hodgkin's lymphomas are categorized on the basis of such factors as morphological characteristics (pathological classification), cell traits ("B cell-like, T cell-like or NK cell-like) or chromosomal and/or genetic information, and are categorized into an extremely diverse range of types according to WHO classification. Follicular lymphomas, which constitute one of the types thereof, have been reported to account of 10 to 15% of malignant lymphomas in Japan, and that number has demonstrated an increasing trend in recent years. Diagnosis of malignant lymphomas is made by assessing the progress of the disease by lymph node biopsy, chest X-ray, computerized tomography (CT), magnetic resonance imaging (MRI), gallium (Ga) scintigraphy or positron emission tomography (PET) and the like. Although blood tests are also used, such as measurement of lactose dehydrogenase (LDH), C-reactive protein (CRP) or soluble interleukin-2 (IL-2) receptors, none of these are diagnostic markers specific for malignant lymphomas, thereby creating a need for a diagnostic marker specific for malignant lymphomas.

Although there have been reports indicating that the concentrations at which autotaxin is present in human tissues or body fluids fluctuate due to various diseases, analyses of the causative relationship with various diseases have not been conducted since there has been no method for quantification thereof. According to the present invention, concentrations of autotaxin in serum and other human body fluids are able to be quantified easily, in a short period of time and with high reliability. This measurement method has made it possible to identify the relationships between autotaxin concentrations and various diseases that were heretofore unable to be determined through the use of measurement reagents. Moreover, as a result of further conducting extensive studies using this measurement system, testing for malignant lymphomas, and particularly follicular lymphomas, for which diagnosis was previously both complex and difficult due to the absence of a serum marker, has also become possible.

The inventors of the present invention were able to accurately quantify human autotaxin by constructing an immunochemical measurement system using antibody to human autotaxin without being affected by intrinsic substances that cause problems during measurement of enzyme activity and without requiring pretreatment of the specimen. As a result of conducting extensive studies using this measurement reagent, it was found that serum autotaxin concentrations exhibit high values in malignant lymphoma patients, and particularly in follicular lymphoma patients, thereby making it possible to provide a reagent capable of detecting or facilitating detection of follicular lymphomas. In addition, this reagent also enables detection or facilitates detection of follicular lymphomas by measurement of the lysophospholipase D enzyme activity of autotaxin, although inferior in terms of complexity and accuracy.

Thus, the present application also includes the following inventions:
(11) a method for detecting malignant lymphoma, comprising: measuring the concentration of autotaxin in a human specimen, and judging to be a malignant lymphoma in the case that the measured value exhibits a significantly higher value than normal values comprised of measured values from normal healthy subjects;
(12) the method of (11), wherein the malignant lymphoma is a follicular lymphoma;
(13) the method of (11) or (12), wherein the autotaxin of (11) is full-length autotaxin, partially cleaved autotaxin or partially genetically mutated autotaxin;
(14) the method described in any of (11) to (13), wherein the specimen of (11) is a human blood component, such as whole blood, blood cells, serum or plasma, or human cells or tissue extract;
(15) the method described in any of (11) to (14), wherein the method for measuring autotaxin concentration of (11) is an immunochemical measurement method using antibody;
(16) the method described in any of (11) to (15), wherein the antibody of (15) is a monoclonal antibody;
(17) the method described in any of (11) to (16), wherein the concentration of autotaxin in the specimen is measured by contacting the antibody of (15) with the specimen, and detecting antibody bound or unbound to the specimen;
(18) the method described in any of (11) to (16), wherein the concentration of autotaxin in the specimen is measured by contacting the antibody of (14) with the specimen and detecting autotaxin bound or unbound to the antibody;
(19) the method described in any of (11) to (18), wherein the method described in any of (15) to (18) is a competitive method using an enzyme label, isotope label or fluorescent label, a homogeneous measurement method using a sandwich method or fluorescent polarization method, or a binding measurement method using a surface plasmon resonance analysis method;
(20) a malignant lymphoma detecting reagent using as the principle thereof the method described in any of (11) to (19);
(21) a detecting method comprising measurement of lysophospholipase D activity possessed by autotaxin using the method described in any of (11) to (14), and judging to be a malignant lymphoma in the case that the measured value exhibits a significantly higher value than normal values comprised of measured values from normal healthy subjects; and
(22) a method for detecting lymphoma comprising measurement of autotaxin in a malignant lymphoma testing reagent specimen using as the principle thereof the detecting method of (21).

According to the aforementioned inventions, malignant lymphomas, and particularly follicular lymphomas, can be detected by quantifying the concentration of autotaxin in a human specimen with a quantification reagent using a monoclonal antibody specific to human autotaxin without requiring pretreatment of the autotaxin in the human specimen. The use of this immunoassay reagent makes it possible to provide a reagent enabling quantification of human autotaxin in a short period of time and without being affected by intrinsic measurement-inhibiting factors or competitive enzymes contained in specimens. In addition, although inferior to the aforementioned immunochemical quantification method in terms of complexity and accuracy, the aforementioned inventions also allow assessment of malignant lymphomas and are able to provide a reagent for that purpose even in the case of measurement of lysophospholipase D enzyme activity.

### BRIEF DESCRIPTION OF THE DRAWINGS

Fig. 1 shows the results of SDS-PAGE and Western blotting using anti-autotaxin peptide antibody for a purified polyhistidine- labeled human autotaxin product.
Fig. 2 shows antibody reactivity during direct binding of human autotaxin to an immunoplate.
Fig. 3 shows the reactivity of anti-human autotaxin antibody bound to an immunoplate by means of anti-rat IgG antibody with human autotaxin present in a solution.
Fig. 4 shows absorption of lysophospholipase D activity of human autotaxin in serum by antibody.
Fig. 5 shows absorption of lysophospholipase D activity of autotaxin in animal serum by antibody.
Fig. 6 shows the results of SDS-PAGE and Western blotting using anti-autotaxin peptide antibody for a purified full-length human autotaxin product.
Fig. 7 shows the reactivity of anti-human autotaxin monoclonal antibody with human autotaxin using a double enzyme sandwich method.
Fig. 8 shows the reactivity in a sandwich ELISA assay system by a combined evaluation using a group of obtained anti-human autotaxin antibodies.
Fig. 9 is a graph showing the relationship between optical absorbance at 450 nm and autotaxin concentration when measuring six standards having known human autotaxin concentrations (0, 0.34, 0.675, 1.35, 2.70 and 5.40 µg/mL) using a double-antibody two-step sandwich ELISA method.
Fig. 10 shows the correlation between human autotaxin concentration and lysophospholipase D activity as determined by a double-antibody two-step sandwich ELISA method in 42 human serum specimens.
Fig. 11 is a graph showing the relationship between optical absorbance at 450 nm and autotaxin concentration when measuring six standards having known human autotaxin concentrations (0, 0.34, 0.675, 1.35, 2.70 and 5.40 µg/mL) using a double-antibody one-step sandwich ELISA method.
Fig. 12 shows the correlation between human autotaxin concentration and lysophospholipase D activity as determined by a double-antibody one-step sandwich ELISA method in 42 human serum specimens.
Fig. 13 shows the results of verifying autotaxin concentrations versus normal healthy subjects using specimens in which measured values of PSA, CA19-9, CA153 and CA125 as determined using a double-antibody one-step sandwich ELISA method exceeded a cutoff value. The presence of a significant difference versus the normal healthy subjects was indicated with p<0.001 for all of the specimen groups.
Fig. 14 shows the results of measuring serum human autotaxin concentrations in chronic liver disease patients and normal healthy subjects using a double-antibody one-step sandwich ELISA method.
Fig. 15 shows the results of measuring lysophospholipase D activity in seminal plasma.
Fig. 16 shows a calibration curve obtained when using six concentration standards. The regression formula was expressed as Log(rate) = aLog(Conc)³ + bLog(Conc)² + cLog(Conc) + d, and the respective constants of the displayed calibration curve consisted of a = -0.12278770, b = -0.30068255, c = 1.26861618 and d = 1.56201600. Rate indicates the amount of 4-methylumbelliferone formed per unit time.
Fig. 17 shows the results of measuring male serum autotaxin concentrations and classifying according to leukemia and malignant lymphoma. Autotaxin (ATX) concentration is plotted on the vertical axis and each disease category is plotted on the horizontal axis. NHS indicates normal healthy subjects, AL indicates acute leukemia, CL indicates chronic leukemia, HD indicates Hodgkin's lymphoma, NHL indicates non-Hodgkin's lymphoma and others indicates other leukemias and lymphomas.
Fig. 18 shows the results of measuring female serum autotaxin concentrations and classifying according to type of leukemia or malignant lymphoma. Autotaxin (ATX) concentration is plotted on the vertical axis and each disease category is plotted on the horizontal axis. NHS indicates normal healthy subjects, AL indicates acute leukemia, CL indicates chronic leukemia, HD indicates Hodgkin's lymphoma, NHL indicates non-Hodgkin's lymphoma and others indicates other leukemias and lymphomas.
Fig. 19 indicates the results of measuring male serum autotaxin concentrations and classifying according to type of non-Hodgkin's lymphoma. Autotaxin (ATX) concentration is plotted on the vertical axis and each type of non-Hodgkin's lymphoma is plotted on the horizontal axis. NHS indicates normal healthy subjects, Burkitt indicates Burkitt's lymphoma, LBL indicates lymphoblastic lymphoma, NHL (MCL) indicates mantle cell lymphoma, NHL (DLBC) indicates diffuse large B-cell lymphoma, and NHL (FL) indicates follicular lymphoma.
Fig. 20 indicates the results of measuring female serum autotaxin concentrations and classifying according to type of non-Hodgkin's lymphoma. Autotaxin (ATX) concentration is plotted on the vertical axis and each type of non-Hodgkin's lymphoma is plotted on the horizontal axis. NHS indicates normal healthy subjects, Burkitt indicates Burkitt's lymphoma, LBL indicates lymphoblastic lymphoma, NHL (MCL) indicates mantle cell lymphoma, NHL (DLBC) indicates diffuse large B-cell lymphoma, and NHL (FL) indicates follicular lymphoma.
Fig. 21 shows the results of a test of the correlation between autotaxin (ATX) concentration and lysophosphatidic acid (LPA) concentration.

### BEST MODE FOR CARRYING OUT THE INVENTION

A nucleic acid molecule encoding human autotaxin gene for preparing an immunizing antigen is obtained by screening a cDNA library or genome library using a polynucleotide probe based on the genetic information of human autotaxin. A cDNA library can be easily prepared by isolating RNA from tissue using a known method, or a commercially available cDNA library may be used. By recombining in an expression vector using the resulting human autotaxin cDNA, antigens can be expressed in various expression systems. In addition, the introduction of a commonly used marker tag such as a polyhistidine tag or Myc tag onto the end of human autotaxin gene is also effective for simplifying the subsequent antigen purification procedure. Although the protein expression system may be *Escherichia coli,* yeast, insect cells or animal cells and the like, an insect cell-baculovirus system is preferable since it enables large-volume expression and expression of protein having a structure similar to that of naturally-occurring form of human autotaxin such as the addition of sugar chains. In the case of expressing autotaxin having a polyhistidine tag, the autotaxin can be easily purified with a metal chelating column and the like. In addition, a c-myc tag and the like allows purification with an anti-c-myc antibody affinity column. These are standard methods for which technologies have been adequately established.

Immunizing antigen used in the present invention enables the production of antibody by any means, such as by immunizing an animal with purified antigen, provided technology thereof has been established. There are no particular limitations on the animal used provided it has the ability to produce antibody, examples of which include conventionally used mammals such as mice, rats or rabbits as well as chickens and the like. For example, in the case of using a mouse, immunization is carried out by administering an emulsion of purified human autotaxin antigen and Freund's complete adjuvant beneath the skin, into the foot pad or into the abdominal cavity. There are also cases in which it is desired to increase antibody titer by repeating additional immunizations with purified antigen and Freund's incomplete adjuvant as necessary. Only antigen is administered to the animal without forming an emulsion with the adjuvant at the time of the final immunization several days prior to cell fusion. Although the administered amount of antigen can be in an order of about 0.4 µg/g of animal body weight, the amount of antigen is that which is not subjected to immunotolerance caused by insufficient or excess antigen.

Preparation of hybridoma cells used in the present invention may employ any method provided a technology thereof has been established, and the fusion means may be any means using electrical fusion or a reagent such as polyethylene glycol. For example, hybridoma cells can be obtained by carrying out cell fusion on B cells of an immunized animal and myeloma cells in the presence of polyethylene glycol followed by selecting antibody-producing cells from HAT medium. The selected hybridoma cells can be used to establish monoclonal antibody-producing hybridoma cells by carrying out monocloning using the limiting dilution method.

A method for selecting a hybridoma used in the present invention is characterized by not carrying out a selection which is performed in a conventional ELISA method, i.e., binding an immunizing antigen to a solid phase, and then performing the selection on the basis of the reactivity for the antigen bound to the surface of the solid phase. The reason for this is that, although screening methods using ELISA in which an antigen is bound to a solid phase allow acquisition of numerous antibodies having reactivity, nearly all of these antibodies are unable to efficiently capture human autotaxin present in serum. In the antibody screening method used in the present invention, a primary antibody capable of capturing antibody contained in the supernatant of a hybridoma cell culture, or other antibody binding factor such as protein A or protein C, is bound to an immunoplate. For example, in the case the objective is to obtain rat antibodies, anti-rat immunoglobulin antibody is bound to an immunoplate. After carrying out blocking treatment to prevent non-specific binding, such as by treating the surface of the immunoplate to which primary antibody is bound with bovine serum albumin and the like, a culture supernatant of hybridoma cells obtained by cell fusion is added to the immunoplate to capture primary antibody. After washing the immunoplate with a buffer such as phosphate-buffered saline (PBS) to remove unreacted substances, recombinant human autotaxin antigen having a polyhistidine tag (polyhistidine-tagged human autotaxin) is added. The polyhistidine-tagged human autotaxin is preferably that which has been confirmed to have lysophospholipase D activity. The polyhistidine-tagged human autotaxin is captured by antibodies in the hybridoma culture supernatant by allowing to react for a fixed amount of time. At this time, the captured polyhistidine-tagged human autotaxin can be presumed to reflect the state that maintains the naturally occurring form in solution in the same manner as in the body, and antibody in the hybridoma culture supernatant capable of capturing the polyhistidine-tagged human autotaxin as a result of this reaction can be expected to be able to capture human autotaxin in a human specimen. Continuing, detection of the polyhistidine-tagged human autotaxin captured by antibody is carried out with enzyme-labeled anti-polyhistidine antibody or an enzyme-labeled probe in the form of HisProbe-HRP (Pierce Biotechnology, Inc., Cat. No. 15165) to the polyhistidine tag of the captured polyhistidine-tagged human autotaxin. Final detection can be carried out using development of color-developing, fluorescent or chemiluminescent substrate for the enzyme, and for example, color can be detected for optical absorbance at a wavelength of 450 nm by using a peroxidase substrate in the form of tetramethylbenzidine (TMB). Nearly all of the antibody groups acquired with this method do not exhibit reactivity in conventional ELISA methods in which human autotaxin is bound directly to an immunoplate. Moreover, a double-antibody sandwich immunoassay system using antibody exhibiting reactivity directly to bound antigen was unable to be constructed. Namely, it was suggested that it is extremely difficult to acquire antibody capable of effectively capturing naturally-occurring form of human autotaxin by an conventional ELISA method in which human autotaxin is bound directly to an immunoplate.

There are no particular limitations on the antibody purification method used in the present invention provided it is a method for which technology has been established. For example, after selecting a target antibody-producing hybridoma, a monoclonal antibody-producing hybridoma is established by limiting dilution followed by recovery of the cell culture supernatant. After concentrating the antibody as necessary by ammonium sulfate precipitation, the monoclonal antibody can be purified by affinity chromatography using a protein A- or protein G-immobilized solid phase support. In addition, the purified antibody can be used to verify the construction of a human autotaxin double-antibody sandwich immunoassay system by labeling with biotin or an enzyme such as alkaline phosphatase. These are standard methods for which technologies have been adequately established.

The method for measuring naturally-occurring form of human autotaxin disclosed in the present invention may be any method provided it allows naturally-occurring form of human autotaxin to be specifically captured and as a result thereof, enables detection of purified antibody-naturally-occurring form of human autotaxin complex. The method is preferably a competitive method utilizing competition between a labeled antigen and naturally-occurring form of human autotaxin for an antibody as is commonly used in immunoassays, or a sandwich method in which a three-component complex is formed between two antibodies having different epitopes and naturally-occurring form of human autotaxin, since these methods are simple and easily universally applied. In the case of binding antibody to a support, there are no particular limitations on the support provided it is used in immunoassays, examples of which include an immunoplate, latex particles, magnetic fine particles, nitrocellulose film and PVDF film. In the case of using a support, human autotaxin can be detected by a method comprising of detecting the enzyme activity of human autotaxin captured by antibody immobilized on the support, or by a method such as surface plasmon resonance in which a specimen is contacted with a chip on which antibody has been immobilized followed by detection of a naturally-occurring form of human autotaxin binding-dependent signal. In addition, human autotaxin can also be quantified with a homogeneous measurement method such as by detecting fluorescence produced as a result of binding between a fluorescent-labeled antibody and naturally-occurring form of human autotaxin. Technologies have been adequately established for these reagents and apparatuses.

A double-antibody sandwich immunoassay method using different epitopes is superior for the aforementioned measurement method from the viewpoints of specificity, sensitivity and universality. Selection of a combination of anti-human autotaxin monoclonal antibodies enabling the construction of a measurement system is carried out using a purified antibody group and a labeled antibody group and verifying whether or not a sandwich assay system can be constructed by using recombinant human autotaxin for the sample. More specifically, purified unlabeled anti-naturally-occurring form of human autotaxin monoclonal antibody is bound to an immunoplate followed by carrying out blocking treatment on the surface of the immunoplate with bovine serum albumin and the like. Continuing, recombinant human autotaxin is added to the immunoplate and captured by the solid phase antibody. After washing the immunoplate with a buffer such as PBS to remove unreacted substances, antibody to labeled naturally-occurring form of human autotaxin is allowed to react to form a sandwich complex of naturally-occurring form of human autotaxin with two types of antibodies. After again washing the immunoplate with a buffer such as PBS to remove unreacted substances, substrate is added in the case of having carried out enzyme labeling for antibody labeling, and enzyme-labeled streptoavidin and the like is allowed to react in cases in which further reaction such as biotin labeling is required. Detection of recombinant human autotaxin captured by the two types of naturally-folding human autotaxin antibodies is then carried out using a color-developing, fluorescent or chemiluminescent substrate for the labeled enzyme. The above experiment is similarly carried out in buffer not containing recombinant human autotaxin for use as a background value. A combination for which the value measured using recombinant human autotaxin is lower than the background value and significantly higher with respect to measured values is used as a human autotaxin immunoassay system candidate.

In order to verify the reliability of the human autotaxin double-antibody sandwich immunoassay system candidate selected in the manner described above, dilution series samples of recombinant human autotaxin and human serum are prepared, and whether or not these samples exhibit dilution factor-dependent reactivity is verified. In the case of an antibody combination for which dependency has been observed, verification is further carried out by quantification of human autotaxin using human serum specimens having known lysophospholipase D activities. Namely, an immunoassay system is selected as a method for measuring naturally-occurring form of human autotaxin by verifying for a correlation between lysophospholipase D enzyme activity and human autotaxin concentration, and selecting an immunoassay system for which a correlation between lysophospholipase D enzyme activity and human autotaxin concentration is observed.

A naturally-occurring form of human autotaxin measurement reagent is prepared using the selected combination of two types of antibodies. In the case of a two-step sandwich measurement reagent, one of the two types of antibodies is bound to a support enabling B/F separation such as an immunoplate or magnetic particles. The binding method may be physical binding using hydrophobic bonds, or chemical binding using a linker reagent capable of crosslinking between two substances. Blocking treatment is carried out on the surface of the support to avoid non-specific binding using bovine serum albumin, skim milk or a commercially available immunoassay blocking agent to obtain a primary reagent. A solution is then prepared containing the other antibody recognizing a different isotope that has been labeled for use as secondary reagent. The antibody label is preferably, for example, an enzyme such as peroxidase or alkaline phosphatase, a detectable substance such as a fluorescent substance, chemiluminescent substance or radioisotope, or a substance for which a specifically binding partner exists such as biotin or avidin. In addition, the secondary reagent solution is preferably a buffer in which an antigen-antibody reaction can be carried out favorably, such as phosphate buffer or Tris-HCl buffer. Measurement of actual specimen consists of contacting the primary reagent and specimen for a fixed amount of time at a fixed temperature. The reaction conditions preferably consist of reacting at a temperature of 4 to 40°C for 5 minutes to 3 hours. Unreacted substances are removed by B/F separation followed by contacting with the secondary reagent for a fixed amount of time and at a fixed temperature to form a sandwich complex. The reaction conditions preferably consist of reacting at a temperature of 4 to 40°C for 5 minutes to 3 hours. Unreacted substances are removed by B/F separation followed by quantifying the label of the labeled antibody and quantifying the concentration of human autotaxin in the sample from a calibration curve prepared by using known concentrations of human autotaxin as standards. In the case of a one-step sandwich measurement reagent, a reagent is prepared in which antibody has been bound to a support followed by blocking treatment in the same manner as a two-step sandwich measurement reagent. A buffer solution containing labeled antibody is added to the antibody-immobilized solid phase support to obtain a reagent. The reagent can also be in the form of a freeze-dried product as necessary. In the case of a one-step reagent, there are many cases in which it is difficult to construct a measurement system due to the occurrences of excesses or shortages of antigen or antibody depending on the balance between the amounts of antigen and antibody used in the one-step reagent. In the present invention, favorable results are obtained in the case of an ELISA method by using 5 to 500 ng and preferably 100 ng of antibody bound to the support and 10 to 100 ng and preferably 10 ng of labeled antibody per well of a 96-well immunoplate. Although the human specimen used for measurement may be serum, plasma, urine, seminal plasma or cerebrospinal fluid and the like, the dilution factor of the specimen used is preferably 0 (undiluted) to 100, and favorable results are obtained by using 10 µL of a five-fold diluted specimen in the case of serum or plasma, and using 100 µL of an undiluted specimen in the case of seminal plasma.

Human autotaxin concentration in a specimen quantified by the measurement reagent according to the present invention exhibits a favorable correlation with lysophospholipase D activity possessed by autotaxin.

Cancer can be diagnosed by measuring human autotaxin in patient serum using the measurement reagent according to the present invention. In a comparison between autotaxin concentrations of specimens in which measured values of prostate cancer marker PSA, colorectal cancer marker CA19-9, breast cancer marker CA153 and ovarian cancer marker CA125 exceeded cutoff levels and autotaxin concentrations of normal healthy subjects not presenting any particular clinical symptoms, significant differences of p<0.001 were demonstrated by all of the cancer patient specimens versus the normal healthy subject group, thus demonstrating results indicating the possibility of diagnosing cancer or assisting in diagnosing cancer. In breast cancer and ovarian cancer in particular, remarkably high values were exhibited versus the normal healthy subjects, and results were obtained indicating that this measurement reagent is effects for diagnosing or assisting in the diagnosis of cancer.

In particular, malignant lymphoma, and especially follicular lymphoma, can be detected by measuring human autotaxin in patient specimens using this measurement reagent. In a comparison between autotaxin concentrations in specimens of patients diagnosed with leukemia or malignant lymphoma and autotaxin concentrations in normal healthy subjects not having any particular clinical symptoms, significantly higher values were demonstrated by the non-Hodgkin's lymphoma patients. In addition, remarkably high values were demonstrated for follicular lymphoma in particular, and results were obtained indicating that this measurement reagent is effective for detecting or facilitating the detection of malignant lymphoma, particularly non-Hodgkin's lymphoma, and more particularly, follicular lymphoma.

Chronic liver disease can also be diagnosed by measuring human autotaxin levels in the serum of chronic liver disease patients using the measurement reagent according to the present invention. As a result of measuring human autotaxin concentrations in specimens of chronic liver disease patients and normal healthy subjects, results were obtained indicating that autotaxin concentrations were higher in the serum of chronic liver disease patients at a significant difference of p<0.0001, thus indicating that this measurement reagent is useful for the diagnosis or assisting in the diagnosis of chronic liver disease. Moreover, as a result of verifying correlations between human autotaxin concentrations and other chronic liver disease markers, correlations were observed between human autotaxin concentration and hyaluronic acid concentration, serum albumin concentration, total bilirubin concentration, platelet count and prothrombin time, and these correlations reflected the degree of chronic liver disease, thus indicating that measurement of serum human autotaxin concentration is extremely effective for diagnosing chronic liver disease.

### EXAMPLES

Although the following indicates examples of the present invention, the present invention is not limited to these examples.

### Example 1: Expression of Recombinant Human Autotaxin

Nucleotide numbers 1 to 2589 (SEQ ID NO: 1) of Autotaxin-t (GenBank accession number L46720) were cloned from a human liver cDNA library using RT-PCR in accordance with ordinary methods. This cDNA was inserted into a baculovirus transfer vector pFASTBac-1 (Invitrogen), and a baculovirus for expressing full-length human autotaxin was prepared using the Bac-to-Bac System (Invitrogen) in accordance with the protocol. The stop codon (TAA nucleotide sequence 2590 to 2592) was removed from the cloned full-length human autotaxin cDNA followed by the addition of 6 histidine residues to prepare a baculovirus for expressing polyhistidine-tagged human autotaxin having polyhistidine in accordance with the protocol. Culture supernatants containing expressed full-length human autotaxin and polyhistidine-tagged human autotaxin were able to be prepared by infecting into sf9 or sf21 and the like in accordance with ordinary methods using these baculoviruses.

### Example 2: Purification of Polyhistidine-Tagged Human Autotaxin

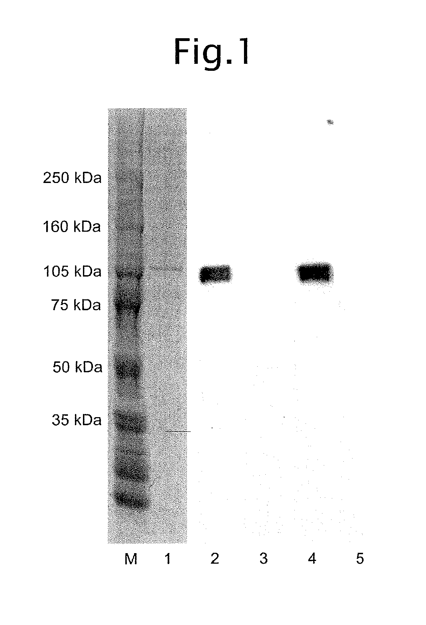

One liter of cells of insect cell line sf21 (5 x 10⁵ cells/mL) were infected with baculovirus for expressing polyhistidine- tagged human autotaxin followed by culturing for 4 days at 28°C. Following completion of culturing, the cells were separated by centrifugal separation (10 minutes at 3000 rpm) followed by removal of cell fragments and other precipitates with a 0.45 µm filter. The recovered culture supernatant was dialyzed with TBS (Tris-buffered saline: 10 mM Tris-HCl, 150 mM NaCl, pH 7.4) and then purified using a metal chelating column packed with BD-TALON Metal Affinity Resin (BD Biosciences, Cat. No. 63501) in accordance with the manual provided. More specifically, the column was packed with 5 mL of resin by volume. 50 mL of a 50 mM CoCl₂ solution were added to the column to bind the cobalt. After washing the column with an aqueous solution containing 300 mM NaCl, the column was equilibrated with a pH 7.7 aqueous solution of 50 mM sodium phosphate and 300 mM NaCl (washing buffer). 50 mL of sample containing polyhistidine-tagged human autotaxin were then added. Unbound substances were washed from the column with 100 mL of washing buffer, and the optical absorbance of solution passing through the column at 280 nm was confirmed to be 0.01 or less. The target substance was eluted from the column with 15 mL of washing buffer containing 10 mM imidazole and then with washing buffer containing 100 mM imidazole. The eluted sample was recovered in 1 mL fractions, and the purity of the polyhistidine-tagged human autotaxin in each fraction was verified by SDS-PAGE. Although the initial fraction was not recovered since it contained numerous impurities, those fractions mainly containing the target polyhistidine-tagged human autotaxin were recovered and mixed and used as purified polyhistidine-tagged human autotaxin antigen. Fig. 1 shows the results of SDS-PAGE and Western blotting using anti-autotaxin peptide antibody for the purified polyhistidine-tagged human autotaxin product. Lane M indicates a molecular weight marker. Lane 1 shows the results of carrying out SDS-PAGE under reducing conditions on the purified antigen at 1 µg/lane followed by staining with CBB. Lanes 2 to 5 show the results of Western blotting. After carrying out SDS-PAGE under reducing conditions on the purified antigen at 0.25 µ3g/lane, the proteins were transferred to a PVDF film, after which the PVDF film was subjected to blocking treatment overnight with TBS containing 3% skim milk. After washing with TBS, the film was immersed in TBS containing 1% Block Ace (Dainippon Pharmaceutical Corp.) and 0.05% Tween 20. 1 µg/mL of rat anti-autotaxin peptide monoclonal antibody (recognizing amino acid sequences 49 to 59 (SEQ ID NO: 2)) were added to Lane 2, while 1 µg/mL of rabbit anti-autotaxin peptide polyclonal antibody (recognizing amino acid sequences 671 to 686 (SEQ ID NO: 3)) were added to Lane 4, followed by allowing to react for 2 hours. (Antibody was not added to the samples of Lanes 3 and 5.) After washing with TBS containing 0.05% Tween 20 (TBST), TBST containing 0.3 µg/mL of alkaline phosphatase-labeled anti-rat IgG antibody (American Qualex, Cat. No. A103AT) and 1% Block Ace was added to the samples of Lanes 2 and 3, while TBST containing 0.3 µg/mL of alkaline phosphatase-labeled anti-rabbit IgG antibody (Zymed) and 1% Block Ace was added to the samples of Lanes 4 and 5. After allowing to react for 2 hours, the film was adequately washed with TBST and chemiluminescence using CDP-STAR (Perkin Elmer) was detected with a photosensitive film. As shown in Lane 1, a single band was confirmed following CBB staining. In addition, according to the results of Western blotting using anti-peptide antibody, the purified antigen was confirmed to be the target polyhistidine-tagged human autotaxin as a result of being detected by two types of antibodies recognizing amino acid sequences 49 to 59 (SEQ ID NO: 2) and amino acid sequences 652 to 666 (SEQ ID NO: 3). Two bands were detected in Western blotting, and results were obtained suggesting differences in sugar chains or decomposition in the culturing and purification steps.

### Example 3: Monoclonal Antibody Production

Seven-week-old female Wistar-Lewis rats were immunized with 250 µg of antigen together with Freund's complete adjuvant into a hind limb under ether anesthesia. One month later, the inguinal lymph nodes and iliac lymph nodes were excised from the rats followed by recovery of B cells. Cell fusion was carried out in accordance with conventional methods with mouse myeloma cell line PAI in the presence of polyethylene glycol, selection was carried out for about 10 days using HAT medium, and target antibodies were selected by screening for antibody-producing hybridomas in accordance with Example 4. Cells in those wells that screened positive were established as hybridomas by monocloning using the limiting dilution method. At that time, after culturing for about 10 days using HT medium, culturing was finally continued with hybridoma medium and the supernatant was recovered to recover the antibody. Medium containing 27.5 mL of NCTC-109 medium (Invitrogen), 5.5 mL of non-essential amino acids (Invitrogen) and 5.5 mL of penicillin/streptomycin/glutamic acid (Invitrogen) in 500 mL of GIT medium (Dainippon Sumitomo Pharma) and being sterilized by filtration was used for the hybridoma cell culturing medium. Medium in which HAT (Sigma-Aldrich Co., HYBRYMAX, Cat. No. H0262) was added to this medium was used as HAT medium, while medium in which HT (Sigma-Aldrich Co., HYBRYMAX, Cat. No. H0137) was added to this medium was used as HT medium.

### Example 4: Hybridoma Screening

Anti-rat immunoglobulin antibody (American Qualex, Cat. No. A103UT) was coated onto a 96-well immunoplate (MaxiSorp; Nalge, NUNC International, Cat. No. 430341) at 250 ng/well. More specifically, anti-rat immunoglobulin antibody was diluted with TBS to prepare a 5 µg/mL solution. This solution was then added to the immunoplate at 50 µL/well and stored overnight at 4°C. Continuing, after washing three times with TBS, TBS solution containing 3% bovine serum albumin (BSA) was added to each well at 250 µL/well followed by allowing to stand for 2 hours at room temperature. After washing three times with TBS, a culture supernatant of hybridoma cells was added at 50 µL/well and allowed to stand for 2 hours at room temperature. After washing six times with TBST, TBS containing 0.6 µg/mL of polyhistidine-tagged human autotaxin, 0.1% Tween 20 and 1% BSA was added at 50 µL/well and allowed to stand for 2 hours at room temperature. After washing six times with TBST, TBS containing 1 µg/mL of HisProbe-HRP (Pierce Biotechnology Inc., Cat. No. 15165), 0.1% Tween 20 and 1% BSA was added at 50 µL/well followed by allowing to stand for 30 minutes at room temperature. After washing six times with TBST, TMB substrate (Kirkegaard & Perry Laboratories, Inc., Cat. No. 50-76-00) was added at 50 µl/well and allowed to stand for 30 minutes at room temperature. The reaction was stopped with 1 N phosphoric acid followed by measurement of optical absorbance at OD 450. At this time, the result of simultaneously carrying out the same procedure with the exception of not containing polyhistidine-tagged human autotaxin was acquired in the form of control data and used as background. Those clones that exhibited reactivity in the presence of polyhistidine-tagged human autotaxin with respect to the control data were selected as positive clones, and monoclonal antibody-producing cell lines were established by limiting dilution.

### Example 5: Antibody Purification and Biotin Labeling

The culture supernatant of monocloned antibody-producing cells was recovered and antibody purification was carried out with HiTrap Protein G HP (GE Healthcare Bioscience Inc., Cat. No. 17-0405-01). The culture supernatant was passed through the aforementioned column after replacing with phosphate-buffered saline (PBS, 10 mM phosphoric acid, 150 mM NaCl, pH 7.4) at a flow rate of 20 mL/min. The column was adequately washed PBS equal to five or more times the volume of the column to remove unbound protein. At that time, unbound protein can be confirmed to no longer be remaining by confirming that the optical absorbance at OD 280 of buffer that has been passed through the column is 0.01 or less. After washing the column, bound antibody was eluted with eluent consisting of 100 mM glycine and having a pH of 2.5. The eluted antibody was promptly added to 1/10 volume of 1 M Tris having a pH of 8 to neutralize together with promptly dialyzing with TBS. A portion of the purified antibody was labeled with biotin using EZ-Link Sulfo-NHS-LC-LC-biotin for antibody evaluation (Pierce Biotechnology, Inc., Cat. No. 21338).

### Example 6: Evaluation of Reactivity of Monoclonal Antibody to Human Autotaxin

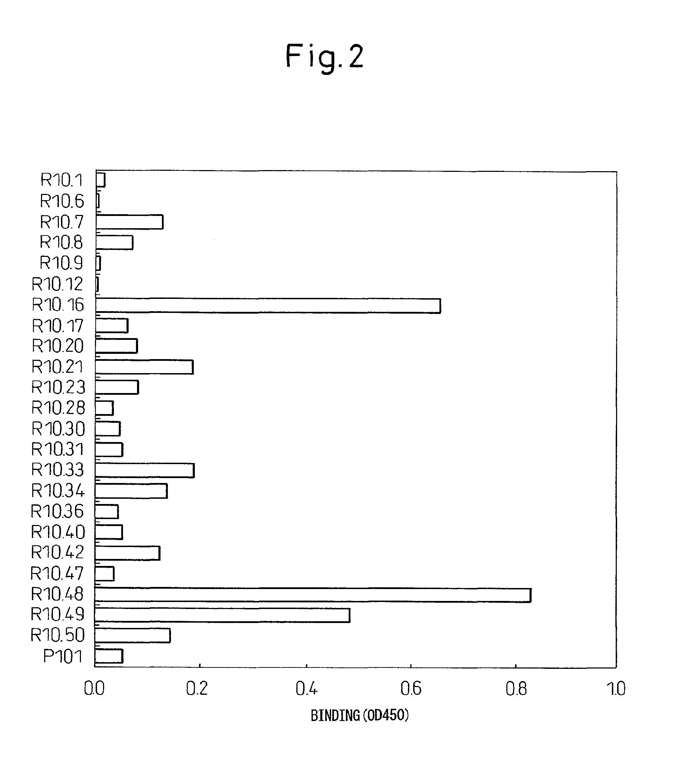

The reactivity of the monoclonal antibody purified according to Example 5 to human autotaxin was verified using two methods consisting of evaluating the reactivity when coating an immunoplate with human autotaxin directly and evaluating the reactivity to human autotaxin present in a solution. An example of coating an immunoplate with human autotaxin is indicated first. Purified recombinant full-length human autotaxin was added to a 96-well immunoplate (MaxiSorp, Nalge NUNC International, Cat. No. 430341) at 50 ng/well (1 µg/mL solution at 50 µL/well) followed by storing overnight at 4°C to coat the immunoplate. Continuing, after washing three times with TBS, TBS solution containing 3% BSA was added to each well at 250 µL/well and allowed to stand for 2 hours at room temperature to carry out blocking. After washing three times with TBS, TBST containing 1 µg/mL of purified antibody and 1% BSA were added at 50 µL/well and allowed to stand for 2 hours at room temperature. After washing six times with TBST, TBST containing 0.3 µg/mL of HRP-labeled goat anti-rat IgG antibody and 1% BSA was added at 50 µL/well and allowed to stand for 2 hours at room temperature. After washing six times with TBST, TMB substrate was added at 50 µL/well followed by allowing to stand for 30 minutes at room temperature. The reaction was stopped with 1 N phosphoric acid, and the results of measuring optical absorbance at OD 450 are shown in Fig. 2. Monoclonal antibody type is plotted on the vertical axis while optical absorbance at 450 nm is plotted on the horizontal axis.

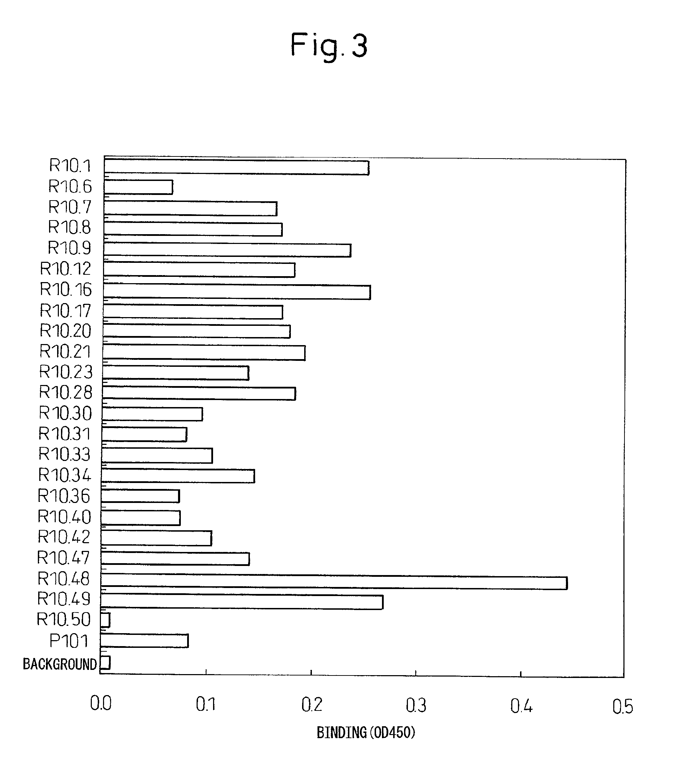

Continuing, the results of verifying the reactivity to human autotaxin in the solution in the manner of Example 4 are indicated. Anti-rat immunoglobulin antibody was coated onto a 96-well immunoplate (MaxiSorp) at 250 ng/well. After washing three times with TBS, a TBS solution containing 3% BSA was added to each well at 250 µL/well followed by allowing to stand for 2 hours at room temperature to carry out blocking. After washing three times with TBS, purified antibody having a concentration of 10 µg/mL was added at 50 µL/well and allowed to stand for 2 hours at room temperature. After washing six times with TBST, TBS containing 0.6 µg/mL of polyhistidine-tagged human autotaxin, 0.1% Tween 20 and 1% BSA was added at 50 µL/well and allowed to stand for 2 hours at room temperature. After washing six times with TBST, TBS containing 1 µg/mL of HisProbe-HRP, 0.1% Tween 20 and 1% BSA was added at 50 µL/well and allowed to stand for 30 minutes at room temperature. After washing six times with TBST, TMB substrate was added at 50 µL/well followed by allowing to stand for 30 minutes at room temperature. The reaction stopped with 1 N phosphoric acid, and the results of measuring optical absorbance at OD 450 are shown in Fig. 3. Monoclonal antibody type is plotted on the vertical axis, while optical absorbance at 450 nm is plotted on the horizontal axis. In addition, reactivity with buffer not containing anti-human autotaxin antibody is shown for the background.

The results of Figs. 2 and 3 indicate that, in the case of an ordinary screening method used during production of monoclonal antibody in the form of an ELISA method comprising binding of antigen, it is extremely difficult to acquire an antibody group capable of efficiently capturing naturally-occurring form of human autotaxin as present in serum as obtained here.

### Example 7: Absorption of Serum Lysophospholipase D Activity by Monoclonal Antibody

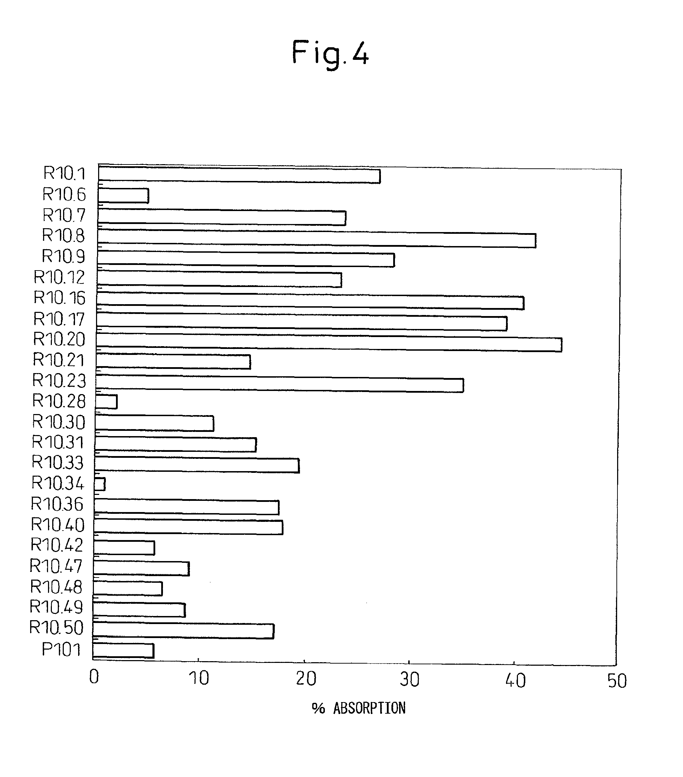

Absorption of human autotaxin in serum by antibody was verified by absorption of serum lysophospholipase D activity. 10 µg of purified anti-human autotaxin antibody were added to 50 µL (50% suspension) of magnetic fine particles on which anti-rat IgG antibody was immobilized in the form of BioMag Goat Anti-Rat IgG Fc (Qiagen, Cat. No. 310144) to prepare anti-human autotaxin-immobilized magnetic fine particles. After washing off unreacted antibody with TBST, 200 µL of human diluted four-fold with TBST were added and allowed to react for 2 hours. After reacting, the magnetic fine particles were removed with a magnet followed by measurement of lysophospholipase D activity in the supernatant. The results of verifying the activity inhibition rate relative to lysophospholipase D activity of human serum treated with anti-rat IgG antibody-immobilized magnetic fine particles in the absence of addition of anti-human autotaxin antibody are shown in Fig. 4. Monoclonal antibody type is plotted on the vertical axis, while activity inhibition rate (absorption rate) is plotted on the horizontal axis. Activity absorption rate indicates the inhibitory activity with respect to residual activity in buffer not containing anti-human autotaxin antibody. Measurement of lysophospholipase D activity was carried out using a slight variation of the method described in FEBS Letters, 571, 197-204, 2004. More specifically, 20 µL of sample were mixed with 20 µL of a substrate solution containing 2 mM lysophosphatidyl choline (14:0-lysophosphatidyl choline), 100 mM Tris-HCl, 500 mM NaCl, 5 mM MgCl₂ and 0.05% Triton X-100 (pH 9.0) followed by allowing to react for 6 hours to overnight at 37°C. Continuing, in order to quantify the choline formed by this enzymatic reaction, 150 µL of a solution R1 comprised of 0.5 mM TOOS (N-ethyl-N-(2-hydroxy-3-sulfopropyl)-3- methylaniline), 10 units/mL horseradish peroxidase, 0.01% Triton X-100 and 100 mM Tris-HCl (pH 8.0) were added and allowed to stand for 5 minutes followed by the addition of 50 µL of a solution R2 comprised of 1 mM 4-aminoantipyrine, 10 units/mL choline oxidase, 0.01% Triton X-100 and 100 mM Tris-HCl (pH 8.0). Thirty minutes later, optical absorbance at 550 nm was measured based on a known concentration of choline chloride and used as the activity value. All of the antibodies except for antibody R10.30 and antibody R10.31 demonstrated absorbance performance, and results were indicated that reflected the binding performance of naturally-occurring form of human autotaxin in solution as determined using an HisProbe in Example 6, while the results did not reflect the result of antigen binding directly to the immunoplate. In the case of antibodies R10.30 and R10.31, differences between absorption performance from serum and measurement using the HisProbe-HRP were presumed to be due to the presence of differences in the structure or form in which it is present between recombinant human autotaxin and human autotaxin present in serum, resulting in these antibodies being unable to recognize human autotaxin in serum.

### Example 8: Confirmation of Reactivity of Monoclonal Antibody to Autotaxins of Other Animal Species

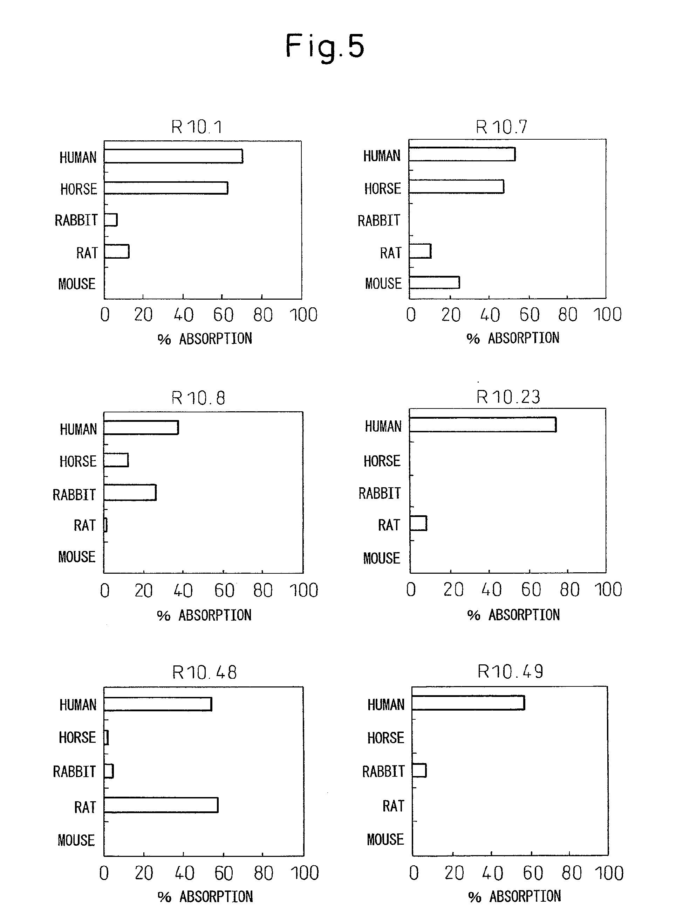

The reactivity to autotaxin in animal serum by the typical antibody purified according to Example 5 was verified according to the absorption of serum lysophospholipase D activity. 10 µg of the biotin-labeled human autotaxin antibody prepared in Example 5 were added to 20 µL (50% suspension) of streptoavidin-immobilized magnetic fine particles in the form of BioMag Streptoavidin (Qiagen, Cat. No. 311711) to prepare anti-human autotaxin-immobilized magnetic fine particles. After washing off unreacted antibody with TBST, 125 µL of animal serum diluted 2.5-fold with TBST were added and allowed to react for 2 hours. After reacting, the magnetic fine particles were removed with a magnet and the lysophospholipase D activity in the supernatant was measured. The results of verifying the activity inhibition rate relative to lysophospholipase D activity of animal serum treated with streptoavidin- immobilized magnetic fine particles in the absence of addition of anti-human autotaxin antibody are shown in Fig. 5 and Table 1. Animal serum type is plotted on the vertical axis, while activity inhibition rate (absorption rate) is plotted on the horizontal axis. Activity absorption rate indicates the inhibitory activity with respect to residual activity in buffer not containing anti-human autotaxin antibody. As exemplified by antibody R10.23, a highly diverse group of antibodies demonstrating cross-reactivity with several types of animal autotaxins was able to be acquired from antibody specific to human autotaxin. Although detailed analyses of the locations of epitopes have been attempted using antigens obtained by expressing autotaxin fragments in *Escherichia coli,* hardly any antibodies demonstrate reactivity with soluble autotaxin fragments, thereby preventing identification of those epitopes. However, since it is predicted that the acquired monoclonal antibody is composed of an antibody group recognizing different epitopes on human autotaxin, results were obtained suggesting the possibility of the construction of a sandwich immunoassay using two antibodies.

**Table 1 Absorption Performance of Lysophospholipase D Activity of Animal Serum Autotaxin by Antibody Less than 10%: - 10-50%: + More than 50%: ++**

| | R10.1 | R10.7 | R10.8 | R10.16 | R10.17 | R10.20 | R10.21 | R10.23 | R10.30 | R10.48 | R10.49 |
|---|---|---|---|---|---|---|---|---|---|---|---|
| Human | ++ | ++ | + | + | ++ | ++ | + | ++ | + | ++ | ++ |
| Horse | ++ | + | + | - | + | + | + | - | - | - | - |
| Rabbit | - | - | + | - | + | + | - | - | - | - | - |
| Rat | + | + | - | - | - | - | - | - | - | ++ | - |
| Mouse | - | + | - | - | - | - | - | - | - | - | - |

### Example 9: Preparation of Human Autotaxin Standard

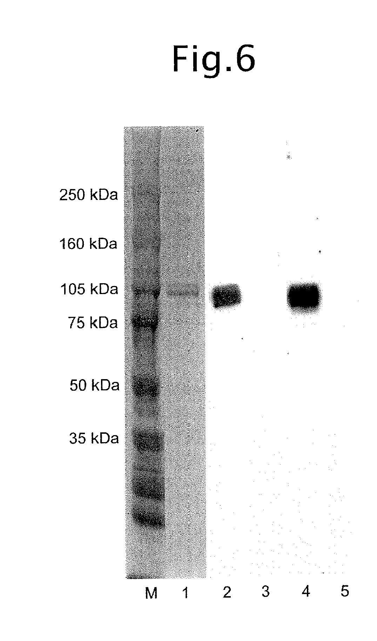

An antibody-immobilized support was prepared using the antibody purified in Example 5 followed by antigen purification and preparation of human autotaxin zero concentration serum in which antigen had been removed from human serum. A standard for use in human autotaxin immunoassay (human autotaxin known concentration sample) was prepared using these materials. More specifically, 25 mg of monoclonal antibody R10.23 purified in accordance with Example 5 was bound to a HiTrap-NHS-Activated 5 mL column (GE Healthcare Bioscience Inc., Cat. No. 17-0717-01) in accordance with the manual. Using this R10.23-bound column, 200 mL of human serum removed of impurities with an 0.8 µm filter were passed through the column at a flow rate of 1 mL/min followed by recovery of the eluate fraction. The human autotaxin in this eluate fraction was confirmed to not demonstrate reactivity in the ELISA measurement method described in Example 6. This product was then used as a base serum for standard preparation and further as a zero concentration standard. Preparation of purified antigen was carried out by using full-length human autotaxin expressed in an insect cell-baculovirus system as the starting material. 1 L of culture supernatant was passed through the R10.23-bound column at a flow rate of 1 mL/min followed by washing off unbound protein with PBS. After confirming the optical absorbance at 280 nm of PBS passed through the column to be 0.01 or less, bound protein was eluted using 100 mM glycine buffer having a pH of 3.5. After neutralizing the eluate by adding one-tenth volume of 1 M Tris (pH 8.0), the eluate was promptly dialyzed with TBS. Fig. 6 shows the results of SDS-PAGE and Western blotting using anti-autotaxin peptide antibody for the purified full-length human autotaxin product. In the drawing, lane M indicates a molecular weight marker. Lane 1 shows the results of carrying out SDS-PAGE under reducing conditions on the purified antigen at 1 µg/lane followed by staining with CBB. Lanes 2 to 5 show the results of Western blotting. Namely, after carrying out SDS-PAGE under reducing conditions on the purified antigen at 0.25 µg/lane, the proteins were transferred to a PVDF film. Following blocking treatment, Lane 2 was detected as rat anti-autotaxin peptide monoclonal antibody (recognizing amino acid sequences 49 to 59 (SEQ ID NO: 2), lane 4 was detected as rabbit anti-autotaxin peptide polyclonal antibody (recognizing amino acid sequences 671 to 686 (SEQ ID NO: 3), and lanes 3 and 5 were detected as background for detecting non-specific binding not containing anti-human autotaxin. Two bands were confirmed for the purified antigen as a result of CBB staining, and two or more bands were detected in the vicinity of a molecular weight of 105 kDa in the same manner as Example 2 in Western blotting using anti-peptide antibody. Thus, results were obtained suggesting differences in sugar chains or decomposition in the culturing and purification steps. Concentration of the purified full-length human autotaxin was measured with the BCA Protein Assay Kit (Pierce Biotechnology, Inc., Cat. No. 23225), and this was used as the human autotaxin concentration. This purified human autotaxin antigen was then added to the aforementioned human autotaxin-free human serum to prepare a known concentration standard.

### Example 10: Selection of Antibody Combination for Sandwich ELISA Measurement System

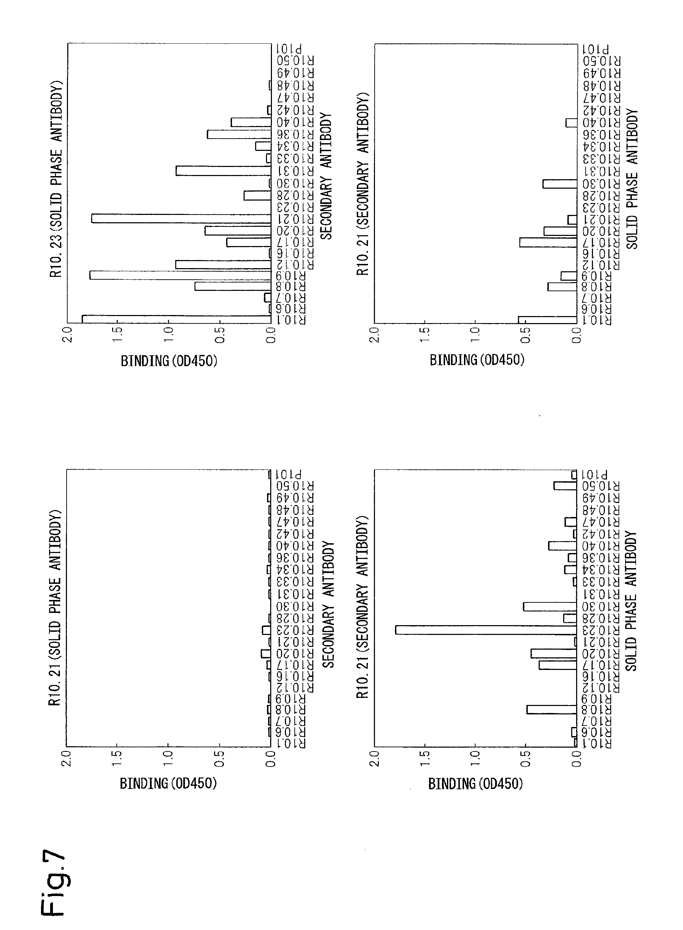

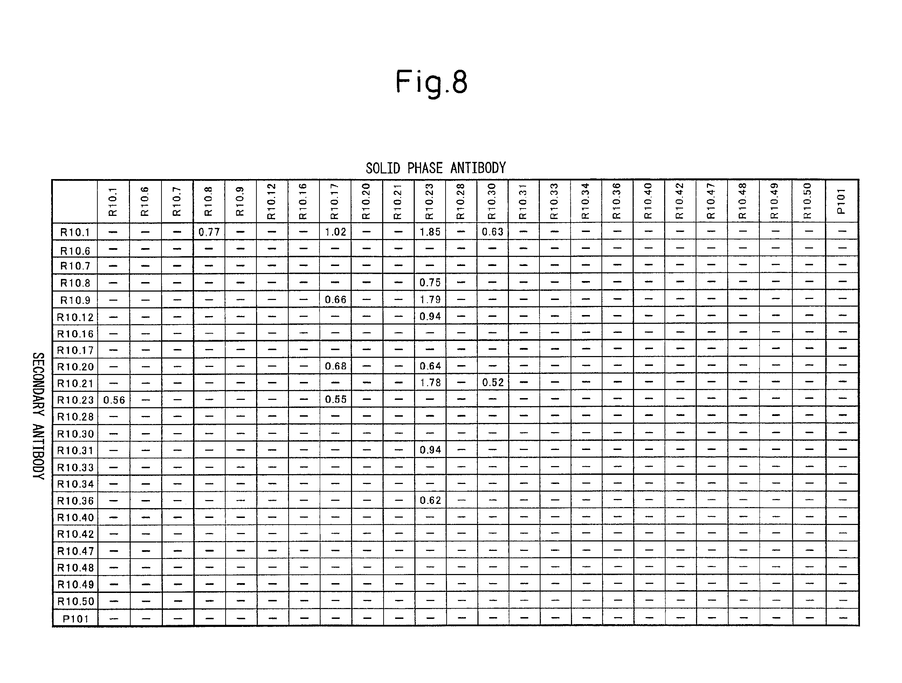

Combinations of antibodies enabling the construction of a sandwich ELISA measurement system were evaluated using purified antigen and biotin-labeled antibody. A TBS solution containing 2 µg/mL of purified antibody were added to a 96-well immunoplate (Nunc) at 50 µL/well to bind the purified antibody to the plate overnight at 4°C. After washing three times with TBS, the recombinant full-length human autotaxin purified according to Example 9 was diluted to 100 ng/mL of purified human autotaxin with ELISA assay buffer (TBS containing 3% BSA, 10 mM MgCl₂ and 0.1% Tween 20) and added at 50 µL/well. After allowing to stand for 2 hours at room temperature, the plate was washed four times with TBST followed by the addition of ELISA assay buffer containing 0.8 µg/mL of biotin-labeled antibody-at 50 µL/well. After allowing to stand for 2 hours at room temperature, the plate was washed four times with TBST followed by the addition of ELISA assay buffer containing HRP-labeled streptoavidin (Zymed) diluted 1000-fold at 50 µL/well. After allowing to stand for 1 hour at room temperature, the plate was washed six times with TBST followed by the addition of TMB substrate at 50 µL/well. After allowing to stand for 10 minutes at room temperature, the reaction was stopped with 1 N phosphoric acid followed by measurement of optical absorbance at OD 450. Typical examples of the results are shown in Fig. 7. In the case of antibodies R10.21 and R10.23, the results of coating each antibody onto an immunoplate as the primary antibody followed by allowing to react with human autotaxin and then detecting reactivity by double-antibody sandwich ELISA using another antibody groups are shown in the top half of the drawing (results when coating R10.21 shown on the left and R10.23 shown on the right onto the plate). In addition, the results of similarly coating acquired antibody groups onto an immunoplate as the primary antibodies followed by detecting reactivity using R10.21 and R10.23 as secondary antibodies are shown in the bottom half of the drawing (results when using R10.21 shown on the left and R10.23 shown on the right as detecting secondary antibodies). In the graphs, the antibodies used are plotted on the horizontal axis (secondary antibodies for the top half of the figure and primary antibodies for the bottom half), while reactivity was determined by optical absorbance at 450 nm is plotted on the vertical axis. As a result of comparing the top and bottom halves of the drawing, there were confirmed to be cases in which reactivity was or was not demonstrated depending on the manner in which the primary antibody and secondary antibody were used even for the same combination of antibodies. Fig. 8 shows a list of reactivities in sandwich ELISA using the acquired anti-human autotaxin monoclonal antibodies. According to these results, those combinations of antibodies that do not exhibit reactivity indicate combinations that cannot be used to construct a sandwich ELISA system, while those combinations that exhibit reactivity suggest that these combinations can be used to construct such a system. The names of primary antibodies bound to the immunoplate are indicated in the horizontal direction, while the names of secondary antibodies are indicated in the vertical direction. In addition, the values in the table indicate optical absorbance at 450 nm, while a "-" sign indicates optical absorbance of 0.5 or less. Among a total of 529 evaluated combinations, there were 16 combinations that exhibited reactivity of 0.5 or more, accounting for about 3% of the total. In addition, combinations using the three types of monoclonal antibodies of R10.16, R10.48 or R10.49, which demonstrated reactivity in ELISA in which the human autotaxin shown in Fig. 2 was bound directly to an immunoplate in correlation with Example 6, did not enable the construction of a sandwich ELISA system. Namely, antibodies acquired by ordinary screening methods in which human autotaxin is bound directly to an immunoplate were suggested to not allow the construction of a sandwich ELISA system or make it extremely difficult to construct such a system, thereby indicating the antibody screening method according to the technique of the present invention to be an extremely effective technique.

### Example 11: Quantification of Human Autotaxin by Two-Step Sandwich ELISA Measurement Method

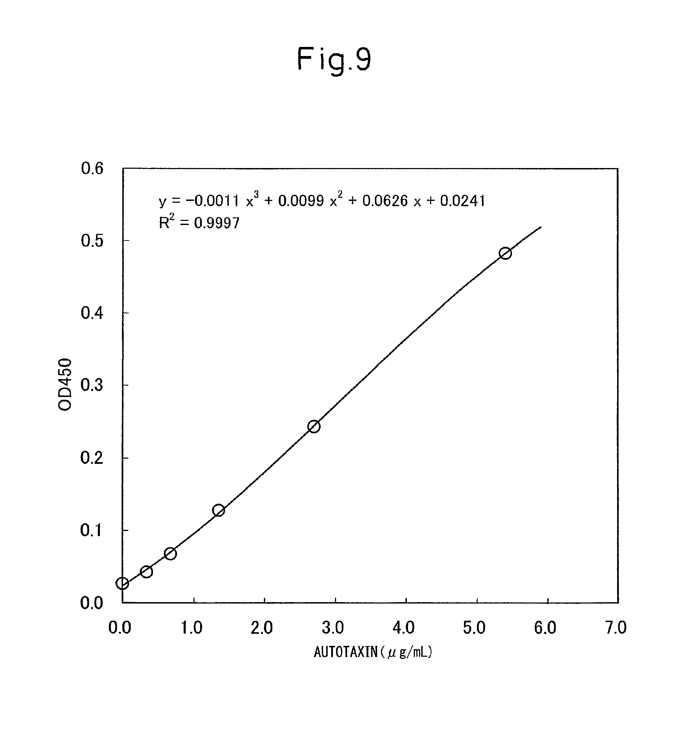

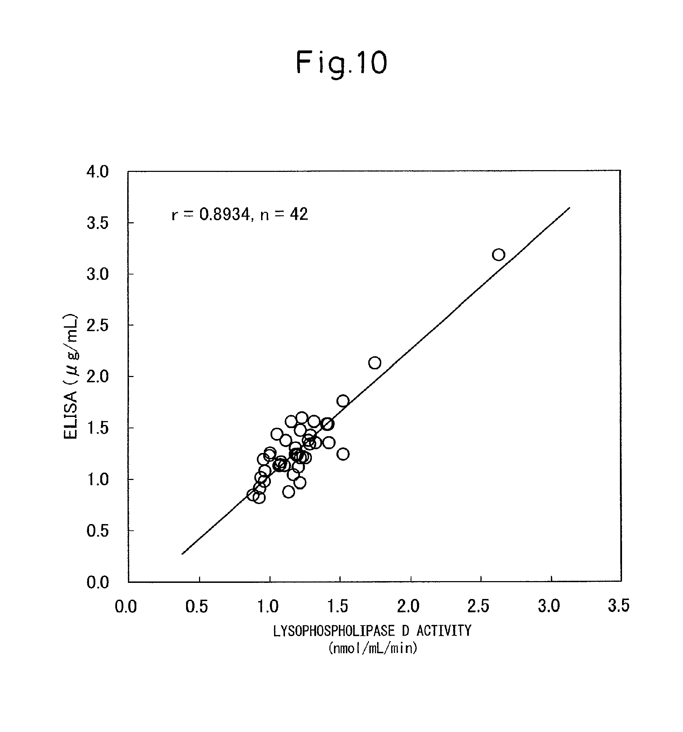

A system for measuring human autotaxin in serum by two-step double-antibody sandwich ELISA was constructed using the combinations of anti-human autotaxin antibodies indicating reactivity in Example 10. The measurement system was verified using R10.23 as solid phase antibody and R10.21 as secondary antibody. The R10.23 solid phase antibody was used after converting the antibody to F(ab)₂ by digestion with pepsin. Using the same procedure as Example 10, R10.23 was added to a 96-well immunoplate (Nunc) at a concentration of 2 µg/mL at 50 µL/well followed by allowing the antibody to bind to the plate overnight at 4°C. After washing three times with TBS, TBS containing 3% BSA was added at 250 µL/well followed by 2 hours of blocking treatment. After washing three times with TBS, the standard prepared in Example 9 and human serum of unknown autotaxin concentration were each diluted by 1/5 with ELISA assay buffer and added to the plate at 50 µL/well. After allowing to react for 2 hours at room temperature, the plate was washed four times with TBST followed by the addition of ELISA assay buffer containing 0.8 µg/mL of biotin-labeled R10.21 at 50 µL/well. After allowing to stand for 2 hours at room temperature, the plate was washed four times with TBST followed by the addition of ELISA assay buffer containing 1000-fold diluted HRP-labeled streptoavidin (Zymed) at 50 µL/well. After allowing to stand for 1 hour at room temperature, the plate was washed six times with TBST followed by the addition of TMB substrate at 50 µL/well. After allowing to stand for 30 minutes at room temperature, the reaction was stopped with 1 N phosphoric acid followed by measurement of optical absorbance at 450 nm. Fig. 9 shows a calibration curve prepared using 6 concentrations of standards having known concentrations of human autotaxin (0, 0.34, 0.675, 1.35, 2.70 and 5.40 µg/mL). Regression of the calibration curve was carried out by third-order regression. Optical absorbance at 450 nm was confirmed to increase dependent on human autotaxin concentration. The concentration of human autotaxin in a specimen was calculated according to optical absorbance at 450 nm obtained from human serum having an unknown autotaxin concentration using this calibration curve. In addition, the lysophospholipase D activity of the human serum used for measurement was determined in accordance with Example 7, and the correlation with human autotaxin concentration was verified. Fig. 10 shows the results for 42 human serum specimens. Lysophospholipase D activity is plotted on the horizontal axis, while human autotaxin concentration is plotted on the vertical axis. Serum human autotaxin concentrations and lysophospholipase D enzyme activity demonstrated a favorable correlation, yielding a correlation coefficient of r = 0.8934, thus indicating that serum autotaxin concentrations can be quantified by the aforementioned sandwich ELISA method.

### Example 12: Quantification of Human Autotaxin by One-Step Sandwich ELISA Measurement Method

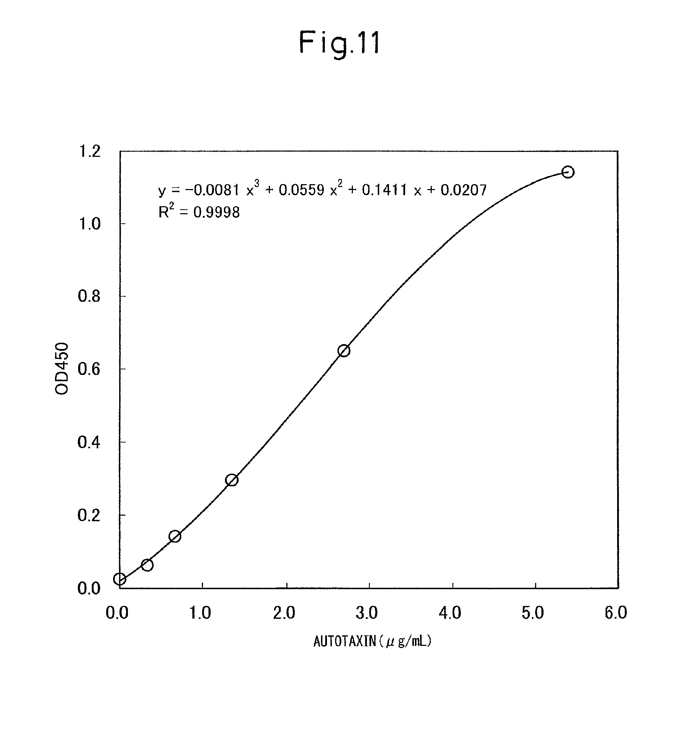

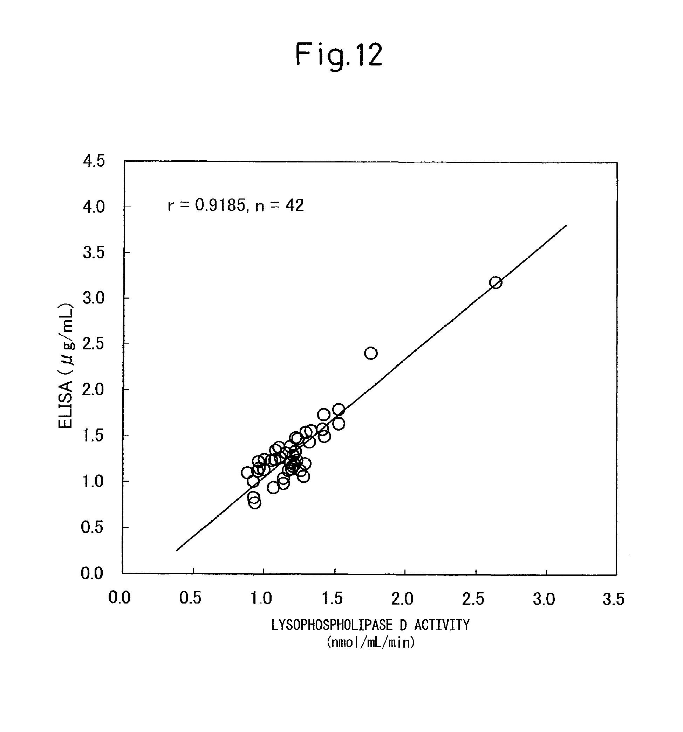

A system for measuring human autotaxin in serum by one-step double-antibody sandwich ELISA was constructed using the combinations of anti-human autotaxin antibodies indicating reactivity in Example 10. The measurement system was verified using R10.23 as solid phase antibody and R10.21 as secondary antibody. The R10.23 solid phase antibody was used after converting the antibody to F(ab)₂. The R10.21 serving as secondary antibody was enzyme-labeled using alkaline phosphatase and Sulfo-SMCC. Using the same procedure as Example 10, R10.23 was added to a 96-well immunoplate (Nunc) at 2 µg/mL followed by allowing the antibody to bind to the plate overnight at 4°C. After washing three times with TBS, TBS containing 3% BSA was added at 250 µL/well followed by 2 hours of blocking treatment. ELISA assay buffer containing 0.57 µg/mL of alkaline phosphatase-labeled R10.21 was added at 50 µL/well and promptly frozen to -40°C. A freeze-dried product was obtained by allowing to stand overnight under reduced pressure to prepare a one-step sandwich ELISA measurement reagent. Measurement was carried out by adding the standard prepared in Example 9 and human serum having an unknown autotaxin concentration diluted by 1/5 with a 0.125% aqueous solution of Tween 20 and adding at 50 µL/well. After allowing to react for 2 hours at room temperature, the plate was washed four times with TBST followed by the addition of TMB substrate at 50 µL/well. After allowing to stand for 30 minutes at room temperature, the reaction was stopped with 1 N phosphoric acid followed by measurement of optical absorbance at 450 nm. Fig. 11 shows a calibration curve prepared using 6 concentrations of standards having known concentrations of human autotaxin (0, 0.34, 0.675, 1.35, 2.70 and 5.40 µg/mL). Regression of the calibration curve was carried out by third-order regression. Optical absorbance at 450 nm was confirmed to increase dependent on human autotaxin concentration. The concentration of human autotaxin in a specimen was calculated according to optical absorbance at 450 nm obtained from human serum having an unknown autotaxin concentration using this calibration curve. In addition, the lysophospholipase D activity of the human serum used for measurement was determined in accordance with Example 7, and the correlation with human autotaxin concentration was verified. Figs. 12 shows lysophospholipase D activity plotted on the horizontal axis versus human autotaxin concentration plotted on the vertical axis. Serum human autotaxin concentrations and lysophospholipase D enzyme activity demonstrated a favorable correlation, yielding a correlation coefficient of r = 0.9185, thus indicating that serum autotaxin concentrations can be quantified by the aforementioned sandwich ELISA method.

### Example 13: Quantification of Human Autotaxin in Cancer Patient Specimens by One-Step Sandwich ELISA Measurement Method and Diagnosis of Cancer

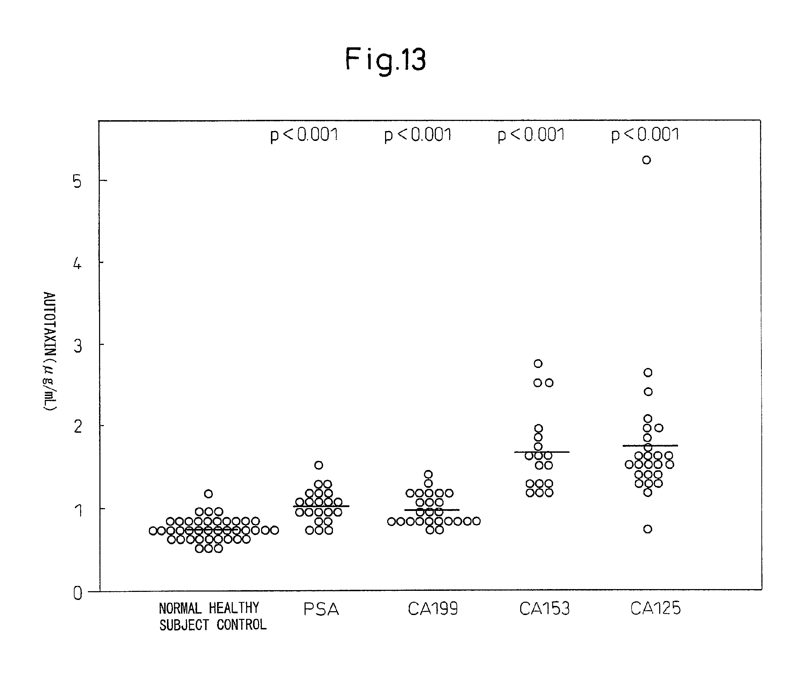

Human autotaxin concentrations in serum specimens from normal healthy subjects and cancer patients were quantified followed by verification of the presence of a significant difference versus the normal healthy subjects. Specimens in which prostate cancer marker PSA (Prostate Specific Antigen), colorectal cancer marker CA19-9, breast cancer marker CA153 and ovarian cancer marker CA125 exceeded cutoff levels were used for the cancer patient serum, and one-step sandwich ELISA was carried out in accordance with Example 12. The levels of each of the cancer markers were measured using the AIA-600II Fully Automated Enzyme Immunoassay System (Tosoh Corp.) along with the respective marker measurement reagents for which manufacturing approval has been obtained for use as in vitro diagnostics. Judgment of a positive result among the cancer patient serum was based on a value of >10 ng/mL for PSA, >38 ng/mL for CA19-9, >23 ng/mL for CA153 and >32 ng/mL for CA125. The results are shown in Fig. 13 and Table 2. Serum autotaxin concentrations in each of the cancer specimen groups demonstrated significantly higher values (p<0.001) than the serum autotaxin concentrations of the normal healthy subjects. On the basis of these results, quantification of the concentration of autotaxin in serum was indicated to be effective for diagnosing cancer.

**Table 2 Results of Verifying Autotaxin Concentrations Versus Normal Healthy Subjects Using Specimens in which CA19-9, CA153 and CA125 Measured Values Exceeded Cutoff Levels Using One-Step Double-Antibody Sandwich ELISA Method Number of measurements, average measured value and standard deviation are shown in the table for each specimen group.**

| | Normal Healthy Subjects | PSA | CA19-9 | CA153 | CA125 |
|---|---|---|---|---|---|
| Number | 40 | 21 | 25 | 17 | 25 |
| Average | 0.73 | 1.01 | 0.97 | 1.68 | 1.75 |
| SD | 0.13 | 0.20 | 0.18 | 0.49 | 0.83 |

### Example 14: Quantification of Human Autotaxin in Chronic Liver Disease Patient Specimens by One-Step Sandwich ELISA Measurement Method and Diagnosis of Chronic Liver Disease

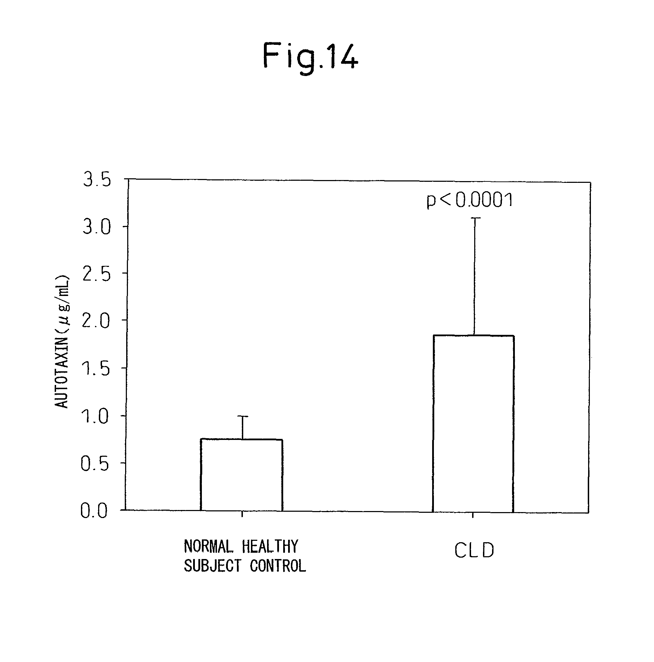

Human autotaxin concentrations in 146 specimens from normal healthy subjects and 29 serum specimens from chronic liver disease (CLD) patients were quantified followed by verification of the presence of a significant difference versus the normal healthy subjects. One-step sandwich ELISA was carried out in accordance with Example 12. The results are shown in Fig. 14. The measured values ± standard deviation of the normal healthy subjects (149 specimens) and chronic liver disease group (27 specimens) were 0.756 ± 0.045 and 0.1866 ± 1.244 ng/mL, respectively, and a significant difference (p<0.0001) was observed. In addition, the relationships between autotaxin concentration and measured values for known liver disease markers in the form of hyaluronic acid, serum albumin, total bilirubin concentration, platelet count and prothrombin time are shown in Table 3 using 17 specimens from chronic liver disease patients. Human autotaxin concentration demonstrated a correlation with all of the known markers. According to these results, quantification of serum autotaxin concentration was indicated to be effective for diagnosing or assisting in the diagnosis of chronic liver disease while also reflecting the degree of that disease.

**Table 3 Results of Measuring Hyaluronic Acid Concentration, Serum Albumin Concentration, Total Bilirubin Concentration, Platelet Count and Prothrombin Time in 17 Chronic Liver Disease Patients and Comparing with Human Autotaxin Concentration of Each Specimen**

| Item | Linear Regression | | Spearman Rank | |
|---|---|---|---|---|
| | Correlation Coefficient r | Reliability p | Correlation coefficient r | Reliability p |
| Hyaluronic acid | 0.764 | 0.0004 | 0.362 | 0.1539 |
| Albumin | -0.776 | 0.0003 | -0.718 | 0.0012 |
| Total bilirubin | 0.821 | 0.0001 | 0.657 | 0.0042 |
| Platelet count | -0.661 | 0.0039 | -0.582 | 0.0143 |
| Prothrombin time | -0.727 | 0.0010 | -0.715 | 0.0013 |

### Example 15: Measurement of Human Seminal Plasma

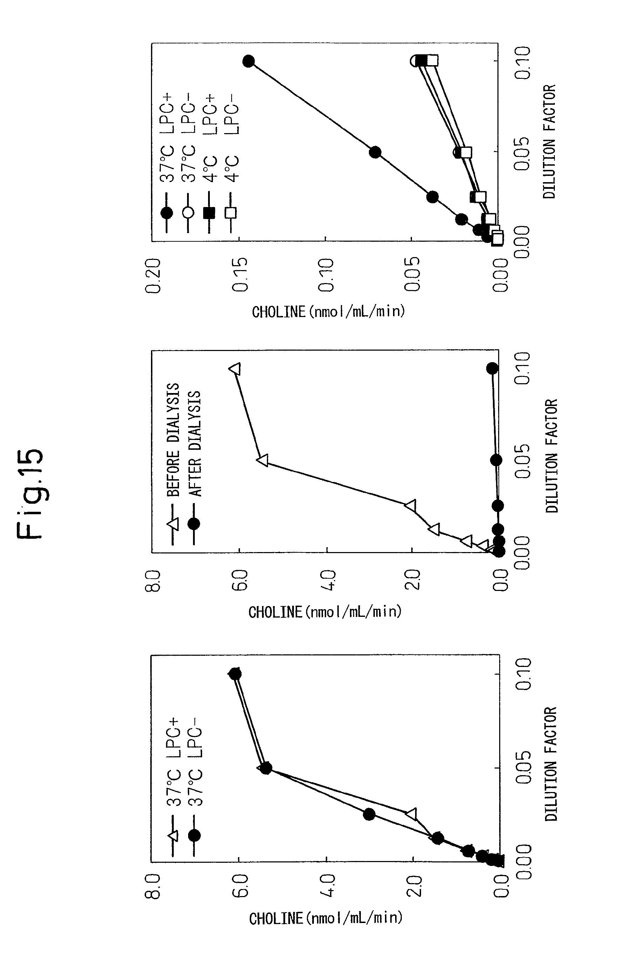

Quantification of autotaxin and measurement of lysophospholipase D activity were carried out in normal healthy subjects. Quantification of autotaxin was carried out using the AIA-600II fully automated enzyme immunoassay system (approval number: 13B3X90002000003). The double-antibody one-step sandwich measurement reagents consisted of using 1.2 µg of R10.23 as solid phase antibody after converting the antibody to F(ab)₂, and using 0.57 µg of alkaline phosphatase-labeled R10.21 as secondary antibody in the same manner as Example 12, and 50 µL of ELISA assay buffer containing 5% gelatin and 10 mM MgCl₂ were dispensed into a measurement cup followed by freeze-drying overnight. Measurement was carried out with an automated system using these measurement reagents. Measurement conditions consisted of dispensing 20 µL of specimen and 130 µL of 0.1% Triton X-100 solution into a measurement cup and allowing to react for 10 minutes at 37°C. After allowing to react and washing with succinate buffer, 4-methylumbelliferyl phosphate was added followed by decomposition by alkaline phosphatase and measurement of the concentration of 4-methylumbelliferone formed per unit time to carry out quantification of autotaxin. The autotaxin concentration of a normal healthy subject when using a calibration curve prepared using six concentrations of standards having a known human autotaxin concentration (0, 0.34, 0.675, 1.35, 2.70 and 5.40 µg/mL) was 0.164 µg/mL. On the other hand, as a result of measuring lysophospholipase D activity in seminal plasma in accordance with Example 7, as shown in the left graph of Fig. 15, there were no large differences observed between the amounts of choline formed in the presence and absence of the substrate in the form of phosphatidyl choline, and extremely high values were exhibited even in seminal plasma diluted ten-fold. Since high values were exhibited in the absence of substrate, it is presumed that intrinsic choline is being measured. Since choline concentration in the seminal plasma of normal healthy subjects is extremely high at about 40 mM based on these measured values, and since the substrate concentration during measurement of lysophospholipase D activity according to Example 7 is 2 mM, it is necessary to dilute the seminal plasma specimen by about 1000-fold in order to carry out measurement while excluding this choline concentration. Since autotaxin is simultaneously diluted as a result of carrying out this dilution procedure, it is predicted that a considerable amount of time is required to measure enzyme activity. In order to confirm that high levels of lysophospholipase D activity in seminal plasma are the result of intrinsic choline, lysophospholipase D activity after removing low molecular weight substances by adequately dialyzing seminal plasma specimens with TBS for 8 hours are shown in the middle graph of Fig. 15. As a result of dialysis, lysophospholipase D activity in the form of the amount of choline formed, which had previously exhibited high values, decreased to about 1/100 the previous levels. In addition, as a result of measuring lysophospholipase D activity in seminal plasma following dialysis, enzyme activity was able to be confirmed in the presence of substrate versus the absence of substrate as shown in the right graph of Fig. 15. In the absence of substrate, values were equivalent to measured values obtained by stopping and controlling the enzyme reaction at a reaction temperature of 4°C. According to these results, since extremely high concentrations of choline are present in seminal plasma, it is difficult to measure lysophospholipase D activity using the formation of choline as an indicator thereof, and these results demonstrated that this measurement only becomes possible by using the immunoassay method according to the present invention.

### Example 16: Preparation of Autotaxin Measurement Reagent

Anti-human autotaxin monoclonal antibody (R10.23) was physically adsorbed overnight at 37°C to a water-insoluble support (ethylene vinyl alcohol support incorporating ferrite having a particle diameter of about 1.5 mm) to an antibody concentration of 100 ng/support followed by blocking for 4 hours at 40°C with 100 mM Tris buffer containing 1% BSA (pH 8.0) to obtain an antibody-immobilized support. Labeled antibody was prepared by converting anti-human autotaxin monoclonal antibody (R10.21) to F(ab)₂ by treating with pepsin followed by binding alkaline phosphatase using SPDP (N-succinimidyl-3-[2-pyridyldithio]propionate) to obtain enzyme-labeled antibody. Twelve antibody-immobilized supports were placed in a magnetically permeable container (volume: 1.2 mL) followed by the addition of 50 µL of a buffer containing 1 µg/mL of labeled antibody (Tris buffer containing 3% BSA, pH 8.0) to the container and freeze-drying to obtain an autotaxin measurement reagent. The autotaxin measurement reagent was sealed after filling the container with nitrogen and stored at 4°C until the time of measurement.

### Example 17: Preparation of Human Autotaxin Standard

An antibody-immobilized support was prepared using anti-human autotaxin monoclonal antibody (R10.23) followed by antigen purification and preparation of human autotaxin zero concentration serum in which antigen had been removed from human serum. A standard for use in human autotaxin immunoassay (human autotaxin known concentration sample) was prepared using these materials. More specifically, 25 mg of monoclonal antibody R10.23 were bound to a HiTrap-NHS-Activated 5 mL column (GE Healthcare Bioscience Inc., Cat. No. 17-0717-01) in accordance with the instruction attached thereto. Using this R10.23-bound column, 200 mL of human serum removed of impurities with an 0.8 µm filter were passed through the column at a flow rate of 1 mL/min followed by recovery of the eluate fraction. The human autotaxin in this eluate fraction was confirmed to not demonstrate reactivity with the measurement reagent described in Example 16. This product was then used as a base serum for standard preparation and further as a zero concentration standard. Preparation of purified antigen was carried out by using for the material full-length human autotaxin expressed in an insect cell-baculovirus system. 1 L of culture supernatant was passed through the R10.23-bound column at a flow rate of 1 mL/min followed by washing off unbound protein with PBS. After confirming the optical absorbance at 280 nm of PBS passed through the column to be 0.01 or less, bound protein was eluted using 100 mM glycine buffer having a pH of 3.5. After neutralizing the eluate by adding one-tenth volume of 1 M Tris (pH 8.0), the eluate was promptly dialyzed with TBS. The concentration of the purified full-length human autotaxin was measured with a BCA Protein Assay Kit (Pierce Biotechnology, Inc., Cat. No. 23225), and this was used as the human autotaxin concentration. This purified human autotaxin antigen was then added to the aforementioned human autotaxin-free human serum to prepare a known concentration standard.

### Example 18: Evaluation of Autotaxin Measurement Reagent

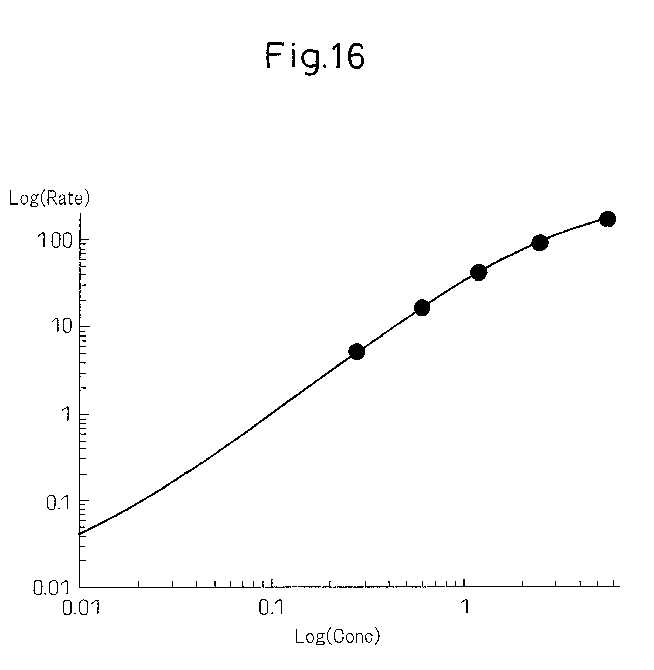

Reagent performance was evaluated using the autotaxin measurement reagent prepared in Example 16 and the standard prepared in Example 17. The AIA-1800 Fully Automated Enzyme Immunoassay System (Tosoh Corp.: Manufacturing and Sales Notification No. 13B3X90002000002) was used for the evaluation apparatus. Six concentrations of standards were used having concentrations of 0, 0.313, 0.625, 1.25, 2.5 and 5.0 µg/mL. The measurement principle of the AIA-1800 Fully Automated Enzyme Immunoassay System consists of automatically dispensing 130 µL of diluent containing 20 µL of human serum specimen and a surfactant into a container containing the autotaxin measurement reagent prepared in Example 16. After going through an antigen-antibody reaction for 10 minutes at a constant temperature of 37°C and washing 8 times with a buffer containing surfactant, 4-methylumbelliferyl phosphate was added to obtain measured values in the form of the concentration of 4-methylumbelliferone formed per unit time (nmol/L·sec). The measured values when measuring the standard are shown in Table 4, while the calibration curved used for that measurement is shown in Fig. 16. In addition, the minimum detectable concentration as calculated from the calibration curve and zero concentration standard (mean + 2 x standard deviation) was 110 ng/mL.

**Table 4**

| Measured Value (nM/s) | Standard Concentration | | | | | |
|---|---|---|---|---|---|---|
| | 0 µg/mL | 0.313 µg/mL | 0.625 µg/mL | 1.25 µg/mL | 2.5 µg/mL | 5.0 µg/mL |
| 1 | 0.038 | 7.444 | 19.937 | 49.888 | 101.739 | 190.031 |
| 2 | 0.041 | 7.473 | 19.026 | 45.471 | 107.094 | 177.043 |
| 3 | 0.040 | 6.942 | 19.241 | 47.992 | 103.751 | 175.219 |
| 4 | 0.040 | 6.325 | 19.850 | 50.465 | 101.417 | 186.175 |
| 5 | 0.039 | 8.248 | 20.577 | 46.465 | 97.992 | 182.891 |
| Mean | 0.040 | 7.286 | 19.726 | 48.056 | 102.399 | 182.272 |
| %CV | 2.879 | 9.770 | 3.114 | 4.460 | 3.264 | 3.392 |

### Example 19: Reproducibility Test of Autotaxin Measurement Reagent

In order to verify the reproducibility of results obtained with the autotaxin measurement reagent, a reproducibility test was carried out with 3 control specimens using the calibration curve prepared in Example 18. Repeatability was assessed by repeatedly measuring the specimens 10 times followed by calculation of the coefficient of variation (%CV = standard deviation/mean x 100). The results are shown in Table 5. In addition, the specimens were also measured every few days followed by calculating the variation from the measured values obtained on day 0 as well as the coefficient of variation of all measured values. Those results are shown in Table 6.

**Table 5 Results of Repeatability Test (µg/mL)**

| | Control Serum L | Control Serum M | Control Serum H |
|---|---|---|---|
| Measured Value 1 | 0.589 | 1.223 | 4.058 |
| Measured Value 2 | 0.619 | 1.261 | 4.18 |
| Measured Value 3 | 0.594 | 1.276 | 3.997 |
| Measured Value 4 | 0.592 | 1.204 | 4.08 |
| Measured Value 5 | 0.581 | 1.244 | 4.156 |
| Measured Value 6 | 0.574 | 1.211 | 4.083 |
| Measured Value 7 | 0.557 | 1.289 | 4.166 |
| Measured Value 8 | 0.588 | 1.204 | 4.09 |
| Measured Value 9 | 0.567 | 1.258 | 4.137 |
| Measured Value 10 | 0.596 | 1.232 | 4.029 |
| Measured Value 11 | 0.556 | 1.259 | 4.124 |
| Measured Value 12 | 0.596 | 1.271 | 4.269 |
| Measured Value 13 | 0.589 | 1.297 | 4.053 |
| Measured Value 14 | 0.577 | 1.264 | 4.186 |
| Measured Value 15 | 0.614 | 1.231 | 4.082 |
| Measured Value 16 | 0.59 | 1.313 | 4.25 |
| Measured Value 17 | 0.571 | 1.255 | 3.928 |
| Measured Value 18 | 0.588 | 1.313 | 4.049 |
| Measured Value 19 | 0.583 | 1.234 | 4.385 |
| Measured Value 20 | 0.613 | 1.25 | 4.059 |
| Mean | 0.59 | 1.25 | 4.12 |
| Standard Deviation | 0.02 | 0.03 | 0.10 |
| %CV | 2.91 | 2.61 | 2.51 |

**Table 6 Results of Inter-Day Reproducibility (µg/mL, mean of four measurements)**

| | Control Serum L | Control Serum M | Control Serum H |
|---|---|---|---|
| Day 0 | 0.779 | 0.804 | 0.975 |
| Day 3 | 0.972 | 0.882 | 1.057 |
| Day 6 | 0.885 | 0.812 | 1.018 |
| Day 13 | 1.186 | 0.908 | 1.052 |
| Day 17 | 1.033 | 0.938 | 1.071 |
| Day 25 | 1.017 | 0.904 | 1.077 |
| Mean | 0.98 | 0.87 | 1.04 |
| Standard Deviation | 0.14 | 0.05 | 0.04 |
| %CV | 14.16 | 6.27 | 3.70 |

Since all coefficients of variation were 10% or less, results obtained with the autotaxin measurement reagent were demonstrated to be reliable.

### Example 20: Measurement of Leukemia and Malignant Lymphoma Specimens

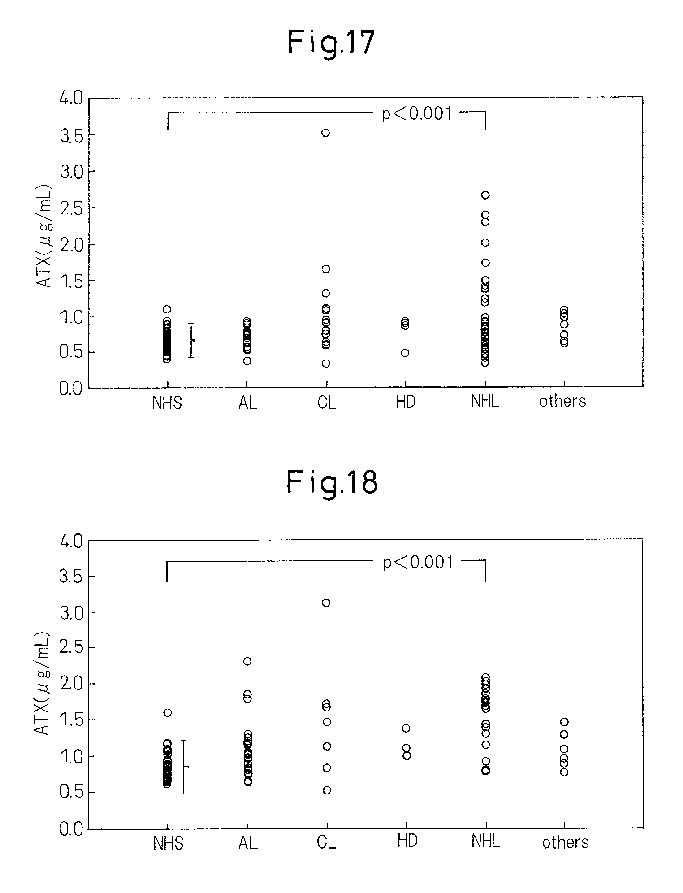

Serum specimens from 120 normal healthy subjects and 215 leukemia and malignant lymphoma patients were measured with the autotaxin measurement reagent using the AIA-600II Fully Automated Enzyme Immunoassay System. The results are shown in Table 7 and Figs. 17 and 18.

**Table 7**

| | NHS | AL | CL | HD | NHL | Others |
|---|---|---|---|---|---|---|
| Men | 74 | 19 | 21 | 4 | 47 | 9 |
| Women | 45 | 33 | 7 | 4 | 20 | 6 |

| | | | | | | |
|---|---|---|---|---|---|---|
| NHS: Normal healthy subjects AL: Acute leukemia CL: Chronic leukemia HD: Hodgkin's lymphoma NHL: Non-Hodgkin's lymphoma Others: Other leukemias and malignant lymphomas | | | | | | |

Measured values of the normal healthy men yielded a mean of 0.656 µg/mL, standard deviation of 0.121 µg/mL, minimum of 0.401 µg/mL and maximum of 1.088 µg/mL. In addition, measured values of the normal healthy women yielded a mean of 0.852 µg/mL, standard deviation of 0.184 µg/mL, minimum of 0.621 µg/mL and maximum of 1.590 µg/mL. Measurements were carried out on 215 leukemia and malignant lymphoma patients. The target specimens consisted of normal healthy subjects, acute leukemia (acute lymphocytic leukemia, acute myelogenous leukemia), chronic leukemia (chronic lymphocytic leukemia, chronic myelogenous leukemia, prolymphocytic leukemia), Hodgkin's lymphoma, non-Hodgkin's lymphoma (diffuse large B-cell lymphoma, follicular lymphoma, mantle cell lymphoma, Burkitt's lymphoma, lymphoblastic lymphoma) and other leukemias and malignant lymphomas (chronic active EBV infection, myelodysplastic syndrome, multiple myeloma), and the respective numbers of specimens are shown in Table 7. The measurement results are as shown in Figs. 17 and 18, and both men and women demonstrated high values for non-Hodgkin's lymphoma at a significance of p<0.01.

### Example 21: Detailed Evaluation of Non-Hodgkin's Lymphoma

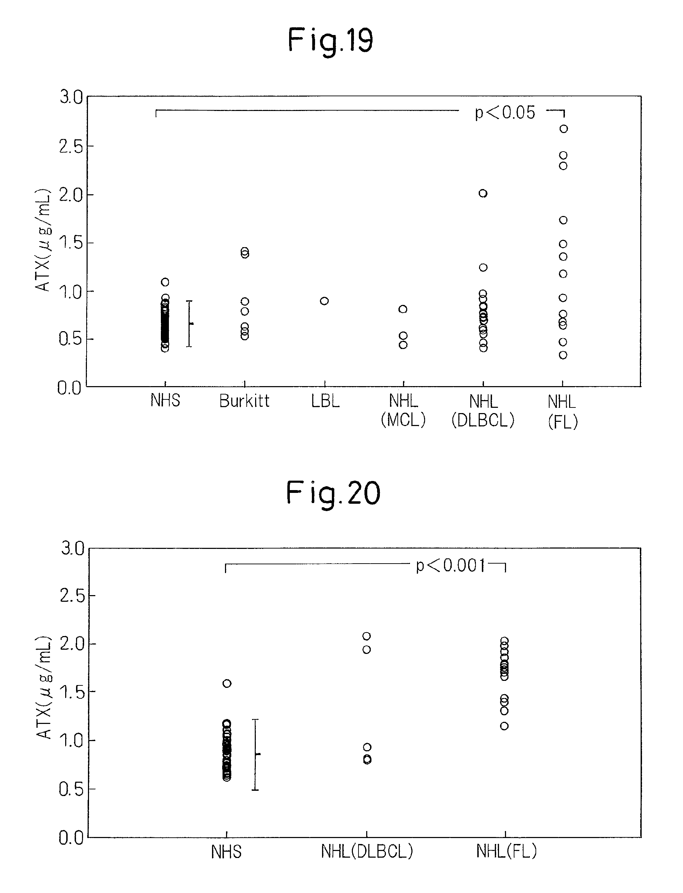

The non-Hodgkin's lymphomas measured in Example 21 were evaluated after classifying in greater detail. The results are shown in Table 8 and Figs. 19 and 20. Both men and women demonstrated high values for follicular lymphomas at a significance of p<0.05 and p<0.01, respectively.

**Table 8**

| | NHS | Burkitt | LBL | MCL | DLBCL | FL |
|---|---|---|---|---|---|---|
| Men | 74 | 7 | 1 | 3 | 23 | 13 |
| Women | 45 | 0 | 0 | 0 | 6 | 14 |

| | | | | | | |
|---|---|---|---|---|---|---|
| NHS: Normal healthy subjects Burkitt: Burkitt's lymphoma LBL: Lymphoblastic lymphoma MCL: Mantle cell lymphoma DLBCL: Diffuse large B-cell lymphoma FL: Follicular lymphoma | | | | | | |

### Example 22: Correlation Study of Autotaxin Concentration and Lysophosphatidic Acid Concentration

Autotaxin has been clearly determined to be involved in the infiltration and metastasis of cancer through the production of a physiologically active lipid in the form of lysophosphatidic acid (LPA). Therefore, autotaxin concentrations were measured to assess their correlation with concentrations of LPA. LPA was measured in accordance with Clin. Chim. Acta., 333, 59-67, 2003. More specifically, 100 mM HEPES buffer (pH 7.6) having the following composition was prepared for use as the measurement reagent.

### (Composition of Measurement Reagent)

20 kU/L lysophospholipase (EC 3.1.1.5)
1.3 kU/L peroxidase
100 kU/L glycerol-3-phosphate oxidase (G3PO: EC 1.1.3.21)
10 kU/L glycerol-3-phosphate dehydrogenase (G3PDH: EC 1.1.1.8)
10 kU/L α-hydroxysteroid dehydrogenase (HSD: EC 1.1.1.50)
10 µM nicotinamide adenine dinucleotide (NADH)
1 mM cholic acid
0.5 mM TOOS
1 mM 4-aminoantipyrine
0.01% Triton X-100

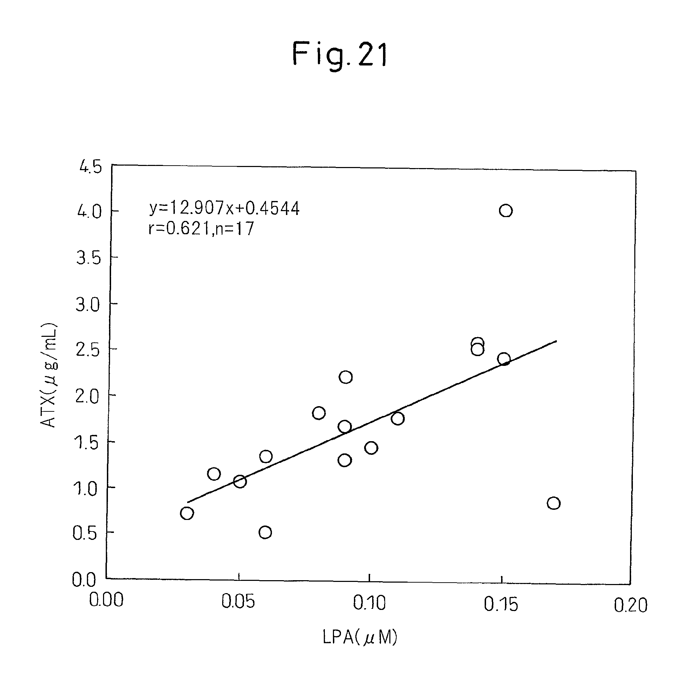

Measurement was carried out by mixing 9 µL of serum and standards having known concentrations of LPA with 240 µL of the aforementioned measurement reagent followed by incubating at 37°C and measuring optical absorbance at 570 nm 7 minutes and 9 minutes later. The amount of increase in the measured values from 7 minutes to 9 minutes (rate value = optical absorbance/ minute) was calculated, and the LPA concentrations in the serum specimens were calculated using a calibration curve prepared from values obtained by measuring the standards. As a result of verifying the correlation between autotaxin antigen concentration in the measured specimen and LPA concentration, a favorable correlation having a correlation coefficient of r = 0.621 was observed as shown in Fig. 21. (Furthermore, a correlation coefficient of r = 0.5 or more can generally be judged to indicate a considerably high correlation.)

Although conventional measurement of LPA concentration was difficult in the clinical setting due to the instability of LPA per se as well as a result of being formed spontaneously in serum following specimen collection, measurement of autotaxin according to the present invention is easy, and a favorable correlation with LPA was obtained.

### INDUSTRIAL APPLICABILITY

The present invention relates to an antibody to naturally-occurring form of human autotaxin contained in a human specimen, a human autotaxin quantification method and quantification reagent using this antibody. The use thereof makes it possible to diagnose cancer and liver disease. Moreover, the present invention makes it possible to detect or facilitate detection of malignant lymphomas, for which definitive serum diagnostic markers were previously not available, particularly non-Hodgkin's lymphoma, exhibiting a high prevalence among Japanese, and more particularly follicular lymphoma, by measuring the concentration of human autotaxin contained in a human specimen by an immunological quantification method.

## Claims

1. A method for detecting chronic liver disease, comprising : measuring the concentration of autotaxin in a human specimen by an immunochemical measurement method using antibody, and judging to be a chronic liver disease in the case that the measured value exhibits a significantly higher value than normal values comprised of measured values from normal healthy subjects.

2. A method for detecting the degree of chronic liver disease comprising : measuring the concentration of autotaxin in a human specimen by an immunochemical measurement method using antibody, and judging the degree on the basis of the measured value.

3. The method according to any of claims 1 or 2,
wherein the specimen is serum, plasma, urine, seminal plasma or cerebrospinal fluid.

4. The method according to any of claims 1 to 3,
wherein antibody is a monoclonal antibody.

5. A reagent for detecting chronic liver disease **characterized by** the principle of the detecting method according to any of claims 1 to 4.


SEQUENCE LISTING

<110>  TOSOH CORPORATION

<120>  Antibody Specific to Native Form of Autotaxin and Screening Method
for the Same

<130>  TYS-T804

<150>  JP 2006-212275
<151>  2006-08-03
<150>  JP 2007-092412
<151>  2007-03-30

<160>  3

<170>  PatentIn version 3.1

<210>  1
<211>  2589
<212>  DNA
<213>  Human

<400>  1
atggcaagga ggagctcgtt ccagtcgtgt cagataatat ccctgttcac ttttgccgtt      60

ggagtcaata tctgcttagg attcactgca catcgaatta agagagcaga aggatgggag     120

gaaggtcctc ctacagtgct atcagactcc ccctggacca acatctccgg atcttgcaag     180

ggcaggtgct ttgaacttca agaggctgga cctcctgatt gtcgctgtga caacttgtgt     240

aagagctata ccagttgctg ccatgacttt gatgagctgt gtttgaagac agcccgtgcg     300

tgggagtgta ctaaggacag atgtggggaa gtcagaaatg aagaaaatgc ctgtcactgc     360

tcagaggact gcttggccag gggagactgc tgtaccaatt accaagtggt ttgcaaagga     420

gagtcgcatt gggttgatga tgactgtgag gaaataaagg ccgcagaatg ccctgcaggg     480

tttgttcgcc ctccattaat catcttctcc gtggatggct tccgtgcatc atacatgaag     540

aaaggcagca aagtcatgcc taatattgaa aaactaaggt cttgtggcac acactctccc     600

tacatgaggc cggtgtaccc aactaaaacc tttcctaact tatacacttt ggccactggg     660

ctatatccag aatcacatgg aattgttggc aattcaatgt atgatcctgt atttgatgcc     720

acttttcatc tgcgagggcg agagaaattt aatcatagat ggtggggagg tcaaccgcta     780

tggattacag ccaccaagca aggggtgaaa gctggaacat tcttttggtc tgttgtcatc     840

cctcacgagc ggagaatatt aaccatattg cagtggctca ccctgccaga tcatgagagg     900

ccttcggtct atgccttcta ttctgagcaa cctgatttct ctggacacaa atatggccct     960

ttcggccctg agatgacaaa tcctctgagg gaaatcgaca aaattgtggg gcaattaatg    1020

gatggactga aacaactaaa actgcatcgg tgtgtcaacg tcatctttgt cggagaccat    1080

ggaatggaag atgtcacatg tgatagaact gagttcttga gtaattacct aactaatgtg    1140

gatgatatta ctttagtgcc tggaactcta ggaagaattc gatccaaatt tagcaacaat    1200

gctaaatatg accccaaagc cattattgcc aatctcacgt gtaaaaaacc agatcagcac    1260

tttaagcctt acttgaaaca gcaccttccc aaacgtttgc actatgccaa caacagaaga 1320

attgaggata tccatttatt ggtggaacgc agatggcatg ttgcaaggaa acctttggat 1380

gtttataaga aaccatcagg aaaatgcttt ttccagggag accacggatt tgataacaag 1440

gtcaacagca tgcagactgt ttttgtaggt tatggcccaa catttaagta caagactaaa 1500

gtgcctccat ttgaaaacat tgaactttac aatgttatgt gtgatctcct gggattgaag 1560

ccagctccta ataatgggac ccatggaagt ttgaatcatc tcctgcgcac taataccttc 1620

aggccaacca tgccagagga agttaccaga cccaattatc cagggattat gtaccttcag 1680

tctgattttg acctgggctg cacttgtgat gataaggtag agccaaagaa caagttggat 1740

gaactcaaca aacggcttca tacaaaaggg tctacagaag agagacacct cctctatggg 1800

cgacctgcag tgctttatcg gactagatat gatatcttat atcacactga ctttgaaagt 1860

ggttatagtg aaatattcct aatgccactc tggacatcat atactgtttc caaacaggct 1920

gaggtttcca gcgttcctga ccatctgacc agttgcgtcc ggcctgatgt ccgtgtttct 1980

ccgagtttca gtcagaactg tttggcctac aaaaatgata agcagatgtc ctacggattc 2040

ctctttcctc cttatctgag ctcttcacca gaggctaaat atgatgcatt ccttgtaacc 2100

aatatggttc caatgtatcc tgctttcaaa cgggtctgga attatttcca aagggtattg 2160

gtgaagaaat atgcttcgga aagaaatgga gttaacgtga taagtggacc aatcttcgac 2220

tatgactatg atggcttaca tgacacagaa gacaaaataa aacagtacgt ggaaggcagt 2280

tccattcctg ttccaactca ctactacagc atcatcacca gctgtctgga tttcactcag 2340

cctgccgaca agtgtgacgg ccctctctct gtgtcctcct tcatcctgcc tcaccggcct 2400

gacaacgagg agagctgcaa tagctcagag gacgaatcaa aatgggtaga agaactcatg 2460

aagatgcaca cagctagggt gcgtgacatt gaacatctca ccagcctgga cttcttccga 2520

aagaccagcc gcagctaccc agaaatcctg acactcaaga catacctgca tacatatgag 2580

agcgagatt 2589

<210> 2
<211> 11
<212> PRT
<213> Human

<400> 2

Asp Ser Pro Trp Thr Asn Ile Ser Gly Ser Cys
1               5                   10

<210> 3
<211> 15
<212> PRT
<213> Human

<400> 3

Cys Val Arg Pro Asp Val Arg Val Ser Pro Ser Phe Ser Gln Asn
1               5                   10                  15